# EUROPEAN PATENT SPECIFICATION

(11) **EP 3 265 468 B1**
(45) Date of publication and mention of the grant of the patent: **10.06.2020**
(21) Application number: 16709516.5
(22) Date of filing: 03.03.2016
(51) Int. Cl.: C07H 1/00, C07H 21/00, C12P 19/34

(54) **A PROCESS FOR THE PREPARATION OF NUCLEIC ACID BY MEANS OF 3'-O-AZIDOMETHYL NUCLEOTIDE TRIPHOSPHATE**
VERFAHREN ZUR HERSTELLUNG VON NUKLEINSÄURE MITTELS 3'-O-AZIDOMETHYL-NUKLEOTID-TRIPHOSPHAT
PROCÉDÉ DE SYNTHÈSE D'ACIDE NUCLÉIQUE PAR L'INTERMÉDIAIRE D'UN 3'-O-AZIDOMÉTHYLNUCLÉOTIDE TRIPHOSPHATE

(30) Priority: 03.03.2015 GB 201503534
(43) Date of publication of application: 10.01.2018
(73) Proprietor: Nuclera Nucleics Ltd, Cambridge Cambridgeshire CB4 0GD (GB)
(72) Inventor: CHEN, Michael C., Cambridge Cambridgeshire CB4 0GD (GB); LAZAR, Radu A., Cambridge Cambridgeshire CB4 0GN (GB); HUANG, Jiahao, Cambridge Cambridgeshire CB4 0GN (GB); MCINROY, Gordon R., Cambridge Cambridgeshire CB4 0GD (GB)
(74) Representative: Gibson, Mark
(86) International application number: PCT/GB2016/050555
(87) International publication number: WO 2016/139477

(56) References cited:
- EP-A1- 2 599 785
- WO-A2-2004/018497
- WO-A2-2010/129656
- CN-A- 103 558 215
- US-A- 5 763 594
- A. F. GARDNER ET AL: "Rapid incorporation kinetics and improved fidelity of a novel class of 3'-OH unblocked reversible terminators", NUCLEIC ACIDS RESEARCH, vol. 40, no. 15, 1 August 2012 (2012-08-01), pages 7404-7415, XP055043174, ISSN: 0305-1048, DOI: 10.1093/nar/gks330
- RAKESH K. PATHAK ET AL: "Copper-Free Click-Chemistry Platform to Functionalize Cisplatin Prodrugs", CHEMISTRY - A EUROPEAN JOURNAL., vol. 20, no. 23, 2 June 2014 (2014-06-02), pages 6861-6865, XP055268818, WEINHEIM, DE ISSN: 0947-6539, DOI: 10.1002/chem.201402573
- LACHLAN S. CAMPBELL-VERDUYN ET AL: "Strain-Promoted Copper-Free "Click" Chemistry for 18F Radiolabeling of Bombesin", ANGEWANDTE CHEMIE INTERNATIONAL EDITION, vol. 50, no. 47, 18 November 2011 (2011-11-18), pages 11117-11120, XP055250606, DE ISSN: 1433-7851, DOI: 10.1002/anie.201105547
- GRIGG R ET AL: "N-Prop-2-ynylmaleimide. Application to Sequential One-pot Rh(I) Catalysed [2+2+2]-Alkyne Cyclotrimerisation-Imine Cycloaddition", TETRAHEDRON, ELSEVIER SCIENCE PUBLISHERS, AMSTERDAM, NL, vol. 56, no. 45, 3 November 2000 (2000-11-03), pages 8967-8976, XP004238552, ISSN: 0040-4020, DOI: 10.1016/S0040-4020(00)00849-8
- ZHANG LIYING ET AL: "Design and synthesis of novel photoaffinity probes for study of the target proteins of oleanolic acid", BIOORGANIC & MEDICINAL CHEMISTRY LETTERS, PERGAMON, AMSTERDAM, NL, vol. 22, no. 2, 7 December 2011 (2011-12-07), pages 1036-1039, XP029121703, ISSN: 0960-894X, DOI: 10.1016/J.BMCL.2011.11.123
- MIRK? PALLA ET AL: "DNA sequencing by synthesis using 3'-O-azidomethyl nucleotide reversible terminators and surface-enhanced Raman spectroscopic detection", RSC ADVANCES, vol. 4, no. 90, 24 September 2014 (2014-09-24), pages 49342-49346, XP055304193, ISSN: 2046-2069, DOI: 10.1039/C4RA08398A

### SEQUENCE LISTING

<110> Nuclera Nucleics Ltd
<120> NOVEL METHOD
<130> NUC-C-P1853PCT
<150> 1503534.8
   <151> 2015-03-03
<160> 8
<170> PatentIn version 3.5
<210> 1
   <211> 517
   <212> PRT
   <213> Sarcophilus harrisii
<400> 1
   Met His Arg Ile Arg Thr Thr Asp Ser Asp His Gly Lys Lys Arg Gln
   1               5                   10                  15
   Lys Lys Met Asp Ala Ile Ser Ser Lys Leu Tyr Glu Ile Lys Phe His
               20                  25                  30
   Glu Phe Val Leu Phe Ile Leu Glu Lys Lys Met Gly Ala Thr Arg Arg
           35                  40                  45
   Thr Phe Leu Met Asp Leu Ala Arg Lys Lys Gly Phe Arg Val Glu Ser
       50                  55                  60
   Glu Leu Ser Asn Ser Val Thr His Ile Val Ala Glu Asn Asn Ser Gly
   65                  70                  75                  80
   Ser Asp Val Leu Ala Trp Leu Glu Ala His Lys Leu Glu Thr Thr Ala
                   85                  90                  95
   His Phe Glu Leu Leu Asp Val Ser Trp Leu Ile Glu Cys Met Lys Val
               100                 105                 110
   Gly Lys Pro Val Asp Thr Lys Gly Lys Tyr Gln Leu Val Glu Ser Ser
           115                 120                 125
   Ile Ala Ser Ala Asn Pro Asp Pro Asn Glu Gly Met Leu Lys Ile Gln
       130                 135                 140
   Ser Pro Ala Met Asn Ala Ile Ser Pro Tyr Ala Cys Gln Arg Arg Thr
   145                 150                 155                 160
   Thr Leu Asn Asn His Asn Gln Arg Phe Thr Asp Ala Phe Glu Ile Leu
   165 170 175
   Ala Lys Asn Tyr Glu Phe Arg Glu Asn His Gly His Cys Leu Thr Phe
   180 185 190
   Leu Arg Ala Thr Ser Val Leu Lys Cys Leu Pro Phe Ala Ile Val Ser
   195 200 205
   Met Lys Asp Ala Glu Gly Leu Pro Trp Ile Gly Asp Glu Val Lys Gly
   210 215 220
   Ile Met Glu Glu Ile Ile Glu Asp Gly Gln Ser Leu Glu Val Gln Ala
   225 230 235 240
   Val Leu Asn Asp Glu Arg Tyr Gln Ala Phe Lys Leu Phe Thr Ser Val
   245 250 255
   Phe Gly Val Gly Leu Lys Thr Ala Glu Lys Trp Tyr Arg Met Gly Phe
   260 265 270
   Arg Thr Leu Ser Lys Ile Gln Ser Asp Lys Ser Leu Lys Phe Thr Lys
   275 280 285
   Met Gln Lys Ala Gly Phe Leu Tyr Tyr Glu Asp Leu Ile Ser Cys Val
   290 295 300
   Ser Lys Ala Glu Ala Asp Ala Val Ser Leu Ile Val Lys Glu Ala Val
   305 310 315 320
   Trp Thr Phe Leu Pro Asp Ala Leu Ile Thr Ile Thr Gly Gly Phe Arg
   325 330 335
   Arg Gly Lys Glu Phe Gly His Asp Val Asp Phe Leu Ile Thr Ser Pro
   340 345 350
   Gly Gly Glu Lys Glu Gln Val Asp Gln Leu Leu Gln Lys Val Thr Asn
   355 360 365
   Leu Trp Glu Lys Gln Gly Leu Leu Leu Tyr Tyr Asp Leu Met Glu Ser
   370 375 380
   Thr Phe Glu Asp Leu Lys Leu Pro Ser Arg Lys Val Asp Ala Leu Asp
   385 390 395 400
   His Phe Gln Lys Cys Phe Leu Ile Leu Lys Leu Tyr Cys Gln Arg Gly
   405 410 415
   Asp Arg Ser Lys Trp Glu Gly Pro Glu Gly Ser Asn Gly Leu Gln Thr
   420 425 430
   Lys Asn Trp Lys Ala Ile Arg Val Asp Leu Val Val Cys Pro Tyr Asp
   435 440 445
   Arg Tyr Ala Tyr Ala Leu Leu Gly Trp Ser Gly Ser Arg Gln Phe Glu
   450 455 460
   Arg Asp Leu Arg Arg Tyr Ala Thr His Glu Lys Lys Met Met Leu Asp
   465 470 475 480
   Asn His Ala Leu Tyr Asp Lys Thr Lys Arg Thr Phe Leu Lys Ala Glu
   485 490 495
   Ser Glu Glu Glu Ile Phe Ser His Leu Gly Leu Glu Tyr Ile Glu Pro
   500 505 510
   Trp Glu Arg Asn Ala
   515
<210> 2
   <211> 494
   <212> PRT
   <213> Lepisosteus oculatus
<400> 2
   Met Leu His Ile Pro Ile Phe Pro Pro Ile Lys Lys Arg Gln Lys Leu
   1 5 10 15
   Pro Glu Ser Arg Asn Ser Cys Lys Tyr Glu Val Lys Phe Ser Glu Val
   20 25 30
   Ala Ile Phe Leu Val Glu Arg Lys Met Gly Ser Ser Arg Arg Lys Phe
   35 40 45
   Leu Thr Asn Leu Ala Arg Ser Lys Gly Phe Arg Ile Glu Asp Val Leu
   50 55 60
   Ser Asp Ala Val Thr His Val Val Ala Glu Asp Asn Ser Ala Asp Glu
   65 70 75 80
   Leu Trp Gln Trp Leu Gln Asn Ser Ser Leu Gly Asp Leu Ser Lys Ile
   85 90 95
   Glu Val Leu Asp Ile Ser Trp Phe Thr Glu Cys Met Gly Ala Gly Lys
   100 105 110
   Pro Val Gln Val Glu Ala Arg His Cys Leu Val Lys Ser Cys Pro Val
           115                 120                 125
   Ile Asp Gln Tyr Leu Glu Pro Ser Thr Val Glu Thr Val Ser Gln Tyr
       130                 135                 140
   Ala Cys Gln Arg Arg Thr Thr Met Glu Asn His Asn Gln Ile Phe Thr
   145                 150                 155                 160
   Asp Ala Phe Ala Ile Leu Ala Glu Asn Ala Glu Phe Asn Glu Ser Glu
                   165                 170                 175
   Gly Pro Cys Leu Ala Phe Met Arg Ala Ala Ser Leu Leu Lys Ser Leu
               180                 185                 190
   Pro His Ala Ile Ser Ser Ser Lys Asp Leu Glu Gly Leu Pro Cys Leu
           195                 200                 205
   Gly Asp Gln Thr Lys Ala Val Ile Glu Asp Ile Leu Glu Tyr Gly Gln
       210                 215                 220
   Cys Ser Lys Val Gln Asp Val Leu Cys Asp Asp Arg Tyr Gln Thr Ile
   225                 230                 235                 240
   Lys Leu Phe Thr Ser Val Phe Gly Val Gly Leu Lys Thr Ala Glu Lys
                   245                 250                 255
   Trp Tyr Arg Lys Gly Phe His Ser Leu Glu Glu Val Gln Ala Asp Asn
               260                 265                 270
   Ala Ile His Phe Thr Lys Met Gln Lys Ala Gly Phe Leu Tyr Tyr Asp
           275                 280                 285
   Asp Ile Ser Ala Ala Val Cys Lys Ala Glu Ala Gln Ala Ile Gly Gln
       290                 295                 300
   Ile Val Glu Glu Thr Val Arg Leu Ile Ala Pro Asp Ala Ile Val Thr
   305                 310                 315                 320
   Leu Thr Gly Gly Phe Arg Arg Gly Lys Glu Cys Gly His Asp Val Asp
                   325                 330                 335
   Phe Leu Ile Thr Thr Pro Glu Met Gly Lys Glu Val Trp Leu Leu Asn
               340                 345                 350
   Arg Leu Ile Asn Arg Leu Gln Asn Gln Gly Ile Leu Leu Tyr Tyr Asp
           355                 360                 365
   Ile Val Glu Ser Thr Phe Asp Lys Thr Arg Leu Pro Cys Arg Lys Phe
       370                 375                 380
   Glu Ala Met Asp His Phe Gln Lys Cys Phe Ala Ile Ile Lys Leu Lys
   385                 390                 395                 400
   Lys Glu Leu Ala Ala Gly Arg Val Gln Lys Asp Trp Lys Ala Ile Arg
                   405                 410                 415
   Val Asp Phe Val Ala Pro Pro Val Asp Asn Phe Ala Phe Ala Leu Leu
               420                 425                 430
   Gly Trp Thr Gly Ser Arg Gln Phe Glu Arg Asp Leu Arg Arg Phe Ala
           435                 440                 445
   Arg His Glu Arg Lys Met Leu Leu Asp Asn His Ala Leu Tyr Asp Lys
       450                 455                 460
   Thr Lys Lys Tyr Leu Lys Lys Lys Thr Thr Asn Asn Tyr Leu Ala Leu
   465                 470                 475                 480
   Asn Asp Val Cys Ser Asp Leu Ser Glu Trp His Tyr Lys Gly
                   485                 490
<210> 3
   <211> 510
   <212> PRT
   <213> Chinchilla lanigera
<400> 3
   Met Asp Pro Leu Gln Ala Ala His Ser Gly Pro Arg Lys Lys Arg Pro
   1               5                   10                  15
   Arg Gln Thr Gly Thr Leu Met Val Ser Ser Pro His Asp Val Arg Phe
               20                  25                  30
   Gly Asp Leu Val Leu Phe Ile Leu Glu Lys Lys Met Gly Thr Thr Arg
           35                  40                  45
   Arg Ala Phe Leu Met Glu Leu Ala Arg Arg Lys Gly Phe Arg Val Glu
       50                  55                  60
   Asn Glu Leu Ser Asp Ser Val Thr His Ile Val Ala Glu Asn Asn Ser
   65                  70                  75                  80
   Gly Asn Asp Val Leu Glu Trp Leu Gln Val Gln Asn Ile Gln Ala Ser
                   85                  90                  95
   Ser Arg Leu Glu Leu Leu Asp Val Ser Trp Leu Ile Glu Cys Met Gly
               100                 105                 110
   Ala Gly Lys Pro Val Glu Met Thr Gly Lys His Gln Leu Leu Val Arg
           115                 120                 125
   Arg Asp Tyr Pro Ala Ser Pro Lys Pro Gly Pro Gln Lys Thr Pro Ser
       130                 135                 140
   Leu Ala Val Gln Lys Ile Ser Glu Tyr Ala Cys Gln Arg Arg Thr Thr
   145                 150                 155                 160
   Leu Asn Asn Cys Asn Cys Ile Phe Thr Asn Ala Phe Glu Ile Leu Ala
                   165                 170                 175
   Glu Asn Cys Glu Phe Arg Glu Asn Glu Ser Ser Tyr Val Thr Tyr Met
               180                 185                 190
   Arg Ala Ala Ser Val Leu Lys Ser Leu Pro Phe Thr Ile Ile Ser Met
           195                 200                 205
   Lys Asp Thr Glu Gly Ile Pro Cys Leu Gly Glu Lys Val Lys Cys Ile
       210                 215                 220
   Ile Glu Glu Ile Ile Glu Asp Gly Glu Ser Ser Glu Val Asn Ala Val
   225                 230                 235                 240
   Leu Asn Asp Glu Arg Tyr Gln Ser Phe Lys Leu Phe Thr Ser Val Phe
                   245                 250                 255
   Gly Val Gly Leu Lys Thr Ser Glu Lys Trp Phe Arg Met Gly Phe Arg
               260                 265                 270
   Ser Leu Asn Lys Ile Lys Ser Asp Lys Ser Leu Lys Phe Thr Arg Met
           275                 280                 285
   Gln Lys Ala Gly Phe Leu Tyr Tyr Glu Asp Leu Val Ser Cys Val Thr
       290                 295                 300
   Arg Ala Glu Ala Glu Ala Val Ser Met Leu Val Lys Glu Ala Val Trp
   305                 310                 315                 320
   Ala Phe Leu Pro Gly Ala Phe Ile Ser Met Thr Gly Gly Phe Arg Arg
                   325                 330                 335
   Gly Lys Glu Ile Gly His Asp Val Asp Phe Leu Ile Thr Ser Pro Glu
               340                 345                 350
   Val Thr Glu Asp Glu Lys Gln Gln Leu Leu His Lys Val Ile Ser Leu
   355 360 365
   Trp Glu Lys Lys Gly Leu Leu Leu Tyr Ser Asp Leu Val Glu Ser Thr
   370 375 380
   Phe Glu Lys Leu Lys Leu Pro Ser Arg Lys Val Asp Ala Leu Asp His
   385 390 395 400
   Phe Gln Lys Cys Phe Leu Ile Leu Lys Leu His His Gln Arg Val Asp
   405 410 415
   Ser Asp Lys Ser Pro Gln Gln Gly Gly Lys Thr Trp Lys Ala Ile Arg
   420 425 430
   Val Asp Leu Val Val Cys Pro Tyr Glu Arg Arg Ala Phe Ala Leu Leu
   435 440 445
   Gly Trp Thr Gly Ser Arg Gln Phe Glu Arg Asp Leu Arg Arg Tyr Ala
   450 455 460
   Thr His Glu Arg Lys Met Met Leu Asp Asn His Ala Leu Tyr Asp Lys
   465 470 475 480
   Thr Lys Arg Ile Phe Leu Lys Ala Glu Ser Glu Glu Glu Ile Phe Ala
   485 490 495
   His Leu Gly Leu Asp Tyr Ile Glu Pro Trp Glu Arg Asn Ala
   500 505 510
<210> 4
   <211> 511
   <212> PRT
   <213> Otolemur garnettii
<400> 4
   Met Asp Pro Leu His Met Ala His Ser Gly Pro Arg Lys Lys Arg Pro
   1 5 10 15
   Arg Gln Thr Ala Ala Ser Met Val Ser Thr Pro Gln Asp Ile Lys Phe
   20 25 30
   Arg Asp Leu Val Leu Phe Ile Leu Glu Lys Lys Met Gly Thr Thr Arg
   35 40 45
   Arg Thr Phe Leu Met Glu Leu Ala Arg Thr Lys Gly Phe Arg Val Glu
   50 55 60
   Asn Glu Phe Ser Asp Ser Val Thr His Ile Ile Ala Glu Asn Asn Ser
   65                  70                  75                  80
   Gly Ser Asp Val Leu Glu Trp Ile Gln Val Gln Lys Ile Lys Ala Gly
                   85                  90                  95
   Ser Gln Met Glu Val Leu Asp Val Ser Trp Leu Ile Glu Cys Met Arg
               100                 105                 110
   Ala Gly Lys Pro Val Glu Met Thr Gly Lys His Gln Leu Val Val Arg
           115                 120                 125
   Gly Asp Tyr Ser Pro Ser Pro Asn Pro Ala Pro Gln Lys Thr Pro Pro
       130                 135                 140
   Leu Ala Val Gln Lys Ile Ser Gln Tyr Ala Cys Gln Arg Arg Thr Thr
   145                 150                 155                 160
   Leu Asn Asn Cys Asn His Ile Phe Thr Asp Ala Phe Glu Ile Met Ala
                   165                 170                 175
   Glu Asn Tyr Glu Phe Arg Glu Asn Glu Gly Tyr Ser Ala Ala Phe Met
               180                 185                 190
   Arg Ala Ala Ser Val Leu Lys Ser Leu Pro Phe Thr Ile Ile Ser Met
           195                 200                 205
   Lys Asp Thr Glu Gly Val Pro Cys Leu Gly Asp Asn Val Lys Cys Ile
       210                 215                 220
   Ile Glu Glu Ile Ile Glu Glu Gly Glu Ser Ser Glu Val Lys Ala Val
   225                 230                 235                 240
   Leu Asn Asp Glu Arg Tyr Gln Ser Phe Lys Leu Phe Thr Ser Val Phe
                   245                 250                 255
   Gly Val Gly Leu Lys Thr Ser Glu Lys Trp Phe Arg Met Gly Phe Arg
               260                 265                 270
   Thr Leu Ser Lys Ile Arg Ser Asp Lys Ser Leu Arg Phe Thr Arg Met
           275                 280                 285
   Gln Gln Ala Gly Phe Leu Tyr Tyr Glu Asp Leu Val Ser Cys Val Thr
       290                 295                 300
   Arg Ala Glu Ala Glu Ala Val Gly Val Leu Val Lys Glu Ala Val Arg
   305                 310                 315                 320
   Ala Phe Leu Pro Asp Ala Phe Val Thr Met Thr Gly Gly Phe Arg Arg
                   325                 330                 335
   Gly Lys Asn Ile Gly His Asp Val Asp Phe Leu Ile Thr Ser Pro Gly
               340                 345                 350
   Ser Thr Glu Glu Glu Glu Gln Gln Leu Leu His Lys Ile Met Asp Leu
           355                 360                 365
   Trp Glu Lys Lys Gly Leu Leu Leu Tyr Cys Asp Leu Val Glu Ser Thr
       370                 375                 380
   Phe Glu Lys Leu Lys Leu Pro Ser Arg Lys Val Asp Ala Leu Asp His
   385                 390                 395                 400
   Phe Gln Lys Cys Phe Leu Ile Phe Lys Leu His His Gln Arg Val Val
                   405                 410                 415
   Asp Ser Glu Gln Ser Asn Gln Gln Glu Gly Lys Thr Trp Lys Ala Ile
               420                 425                 430
   Arg Val Asp Leu Val Met Cys Pro Tyr Glu Arg Arg Ala Tyr Ala Leu
           435                 440                 445
   Leu Gly Trp Thr Gly Ser Arg Gln Phe Glu Arg Asp Leu Arg Arg Tyr
       450                 455                 460
   Ala Thr His Glu Arg Lys Met Ile Leu Asp Asn His Gly Leu Trp Asp
   465                 470                 475                 480
   Lys Thr Lys Arg Ile Phe Leu Lys Ala Glu Ser Glu Glu Glu Ile Phe
                   485                 490                 495
   Ala His Leu Gly Leu Asp Tyr Ile Glu Pro Trp Glu Arg Asn Ala
               500                 505                 510
<210> 5
   <211> 510
   <212> PRT
   <213> Sus scrofa
<400> 5
   Met Asp Pro Pro Gln Thr Val Pro Ser Ser Pro Arg Lys Lys Arg Pro
   1               5                   10                  15
   Arg Gln Val Gly Ala Ser Met Ala Ser Pro Ala His Asn Ile Lys Phe
               20                  25                  30
   Arg Glu Leu Val Leu Phe Ile Leu Glu Lys Lys Met Gly Thr Thr Arg
           35                  40                  45
   Arg Thr Phe Leu Met Glu Leu Ala Arg Arg Lys Gly Phe Arg Val Glu
       50                  55                  60
   Asn Glu Leu Ser Asp Ser Val Thr His Ile Val Ala Glu Asn Asn Ser
   65                  70                  75                  80
   Gly Ser Glu Val Leu Glu Trp Leu Gln Ala Gln Lys Ile Arg Ala Ser
                   85                  90                  95
   Ser Gln Leu Thr Leu Leu Asp Val Ser Trp Leu Ile Glu Ser Met Gly
               100                 105                 110
   Ala Gly Lys Pro Val Glu Met Thr Gly Lys His Gln Leu Val Val Arg
           115                 120                 125
   Thr Asp Cys Ser Ala Ser Pro Ser Pro Gly Ser Gln Asn Thr Leu Pro
       130                 135                 140
   Pro Ala Val Lys Lys Ile Ser Gln Tyr Ala Cys Gln Arg Arg Thr Thr
   145                 150                 155                 160
   Leu Asn Asn Cys Asn His Ile Phe Thr Asp Ala Phe Glu Val Leu Ala
                   165                 170                 175
   Glu Asn Tyr Glu Phe Arg Glu Asn Glu Thr Phe Cys Leu Ala Phe Met
               180                 185                 190
   Arg Ala Ala Ser Val Leu Lys Ser Leu Pro Phe Thr Ile Ile Ser Met
           195                 200                 205
   Lys Asp Thr Glu Gly Ile Pro Cys Leu Gly Asp Lys Val Lys Cys Val
       210                 215                 220
   Ile Glu Glu Ile Ile Glu Asp Gly Glu Ser Ser Glu Val Lys Ala Val
   225                 230                 235                 240
   Leu Asn Asp Glu Arg Tyr Gln Ser Phe Lys Leu Phe Thr Ser Val Phe
                   245                 250                 255
   Gly Val Gly Leu Lys Thr Ser Glu Arg Trp Phe Arg Met Gly Phe Arg
               260                 265                 270
   Ser Leu Ser Lys Ile Arg Ser Asp Lys Thr Leu Lys Phe Thr Arg Met
           275                 280                 285
   Gln Lys Ala Gly Phe Leu Tyr Tyr Glu Asp Leu Val Ser Cys Val Thr
       290                 295                 300
   Arg Ala Glu Ala Glu Ala Val Gly Val Leu Val Lys Glu Ala Val Gln
   305                 310                 315                 320
   Ala Phe Leu Pro Asp Ala Phe Val Thr Met Thr Gly Gly Phe Arg Arg
                   325                 330                 335
   Gly Lys Lys Met Gly His Asp Val Asp Phe Leu Ile Thr Ser Pro Gly
               340                 345                 350
   Ser Thr Asp Asp Glu Glu Gln Gln Leu Leu Pro Lys Val Val Asn Leu
           355                 360                 365
   Trp Glu Arg Glu Gly Leu Leu Leu Tyr Cys Asp Leu Val Glu Ser Thr
       370                 375                 380
   Leu Glu Lys Ser Lys Leu Pro Ser Arg Asn Val Asp Ala Leu Asp His
   385                 390                 395                 400
   Phe Gln Lys Cys Phe Leu Ile Leu Lys Leu His His Gln Arg Val Asp
                   405                 410                 415
   Ser Gly Met Ser Ser Gln Gln Glu Gly Lys Thr Trp Lys Ala Ile Arg
               420                 425                 430
   Val Asp Leu Val Met Cys Pro Tyr Glu Leu Arg Ala Phe Ala Leu Leu
           435                 440                 445
   Gly Trp Thr Gly Ser Arg Gln Phe Glu Arg Asp Leu Arg Arg Tyr Ala
       450                 455                 460
   Thr His Glu Arg Lys Met Ile Leu Asp Asn His Ala Leu Tyr Asp Lys
   465                 470                 475                 480
   Thr Lys Arg Ile Phe Leu Lys Ala Glu Ser Glu Glu Glu Ile Phe Ala
                   485                 490                 495
   His Leu Gly Leu Asp Tyr Leu Glu Pro Trp Glu Arg Asn Ala
               500                 505                 510
<210> 6
   <211> 509
   <212> PRT
   <213> Bos taurus
<400> 6
   Met Asp Pro Leu Cys Thr Ala Ser Ser Gly Pro Arg Lys Lys Arg Pro
   1               5                   10                  15
   Arg Gln Val Gly Ala Ser Met Ala Ser Pro Pro His Asp Ile Lys Phe
               20                  25                  30
   Gln Asn Leu Val Leu Phe Ile Leu Glu Lys Lys Met Gly Thr Thr Arg
           35                  40                  45
   Arg Asn Phe Leu Met Glu Leu Ala Arg Arg Lys Gly Phe Arg Val Glu
       50                  55                  60
   Asn Glu Leu Ser Asp Ser Val Thr His Ile Val Ala Glu Asn Asn Ser
   65                  70                  75                  80
   Gly Ser Glu Val Leu Glu Trp Leu Gln Val Gln Asn Ile Arg Ala Ser
                   85                  90                  95
   Ser Gln Leu Glu Leu Leu Asp Val Ser Trp Leu Ile Glu Ser Met Gly
               100                 105                 110
   Ala Gly Lys Pro Val Glu Ile Thr Gly Lys His Gln Leu Val Val Arg
           115                 120                 125
   Thr Asp Tyr Ser Ala Thr Pro Asn Pro Gly Phe Gln Lys Thr Pro Pro
       130                 135                 140
   Leu Ala Val Lys Lys Ile Ser Gln Tyr Ala Cys Gln Arg Lys Thr Thr
   145                 150                 155                 160
   Leu Asn Asn Tyr Asn His Ile Phe Thr Asp Ala Phe Glu Ile Leu Ala
                   165                 170                 175
   Glu Asn Ser Glu Phe Lys Glu Asn Glu Val Ser Tyr Val Thr Phe Met
               180                 185                 190
   Arg Ala Ala Ser Val Leu Lys Ser Leu Pro Phe Thr Ile Ile Ser Met
           195                 200                 205
   Lys Asp Thr Glu Gly Ile Pro Cys Leu Gly Asp Lys Val Lys Cys Ile
       210                 215                 220
   Ile Glu Glu Ile Ile Glu Asp Gly Glu Ser Ser Glu Val Lys Ala Val
   225                 230                 235                 240
   Leu Asn Asp Glu Arg Tyr Gln Ser Phe Lys Leu Phe Thr Ser Val Phe
   245 250 255
   Gly Val Gly Leu Lys Thr Ser Glu Lys Trp Phe Arg Met Gly Phe Arg
   260 265 270
   Ser Leu Ser Lys Ile Met Ser Asp Lys Thr Leu Lys Phe Thr Lys Met
   275 280 285
   Gln Lys Ala Gly Phe Leu Tyr Tyr Glu Asp Leu Val Ser Cys Val Thr
   290 295 300
   Arg Ala Glu Ala Glu Ala Val Gly Val Leu Val Lys Glu Ala Val Trp
   305 310 315 320
   Ala Phe Leu Pro Asp Ala Phe Val Thr Met Thr Gly Gly Phe Arg Arg
   325 330 335
   Gly Lys Lys Ile Gly His Asp Val Asp Phe Leu Ile Thr Ser Pro Gly
   340 345 350
   Ser Ala Glu Asp Glu Glu Gln Leu Leu Pro Lys Val Ile Asn Leu Trp
   355 360 365
   Glu Lys Lys Gly Leu Leu Leu Tyr Tyr Asp Leu Val Glu Ser Thr Phe
   370 375 380
   Glu Lys Phe Lys Leu Pro Ser Arg Gln Val Asp Thr Leu Asp His Phe
   385 390 395 400
   Gln Lys Cys Phe Leu Ile Leu Lys Leu His His Gln Arg Val Asp Ser
   405 410 415
   Ser Lys Ser Asn Gln Gln Glu Gly Lys Thr Trp Lys Ala Ile Arg Val
   420 425 430
   Asp Leu Val Met Cys Pro Tyr Glu Asn Arg Ala Phe Ala Leu Leu Gly
   435 440 445
   Trp Thr Gly Ser Arg Gln Phe Glu Arg Asp Ile Arg Arg Tyr Ala Thr
   450 455 460
   His Glu Arg Lys Met Met Leu Asp Asn His Ala Leu Tyr Asp Lys Thr
   465 470 475 480
   Lys Arg Val Phe Leu Lys Ala Glu Ser Glu Glu Glu Ile Phe Ala His
   485 490 495
   Leu Gly Leu Asp Tyr Ile Glu Pro Trp Glu Arg Asn Ala
   500 505
<210> 7
   <211> 12
   <212> DNA
   <213> Artificial
<220>
   <223> Synthetic Oligonucleotide
<400> 7
   cgttaacatatt 12
<210> 8
   <211> 494
   <212> PRT
   <213> Synthetic Sequence
<400> 8
   Met Leu His Ile Pro Ile Phe Pro Pro Ile Lys Lys Arg Gln Lys Leu
   1 5 10 15
   Pro Glu Ser Arg Asn Ser Cys Lys Tyr Glu Val Lys Phe Ser Glu Val
   20 25 30
   Ala Ile Phe Leu Val Glu Arg Lys Met Gly Ser Ser Arg Arg Lys Phe
   35 40 45
   Leu Thr Asn Leu Ala Arg Ser Lys Gly Phe Arg Ile Glu Asp Val Leu
   50 55 60
   Ser Asp Ala Val Thr His Val Val Ala Glu Asn Asn Ser Ala Asp Glu
   65 70 75 80
   Leu Leu Gln Trp Leu Gln Asn Ser Ser Leu Gly Asp Leu Ser Lys Ile
   85 90 95
   Glu Val Leu Asp Ile Ser Trp Phe Thr Glu Cys Met Gly Ala Gly Lys
   100 105 110
   Pro Val Gln Val Glu Ala Arg His Cys Leu Val Lys Ser Cys Pro Val
   115 120 125
   Ile Asp Gln Tyr Leu Glu Pro Ser Thr Val Glu Thr Val Ser Gln Tyr
   130 135 140
   Ala Cys Gln Arg Arg Thr Thr Met Glu Asn His Asn Gln Ile Phe Thr
   145 150 155 160
   Asp Ala Phe Ala Ile Leu Ala Glu Asn Ala Glu Phe Asn Glu Ser Glu
                   165                 170                 175
   Gly Pro Cys Leu Ala Phe Met Arg Ala Ala Ser Leu Leu Lys Ser Leu
               180                 185                 190
   Pro His Ala Ile Ser Ser Ser Lys Asp Leu Glu Gly Leu Pro Cys Leu
           195                 200                 205
   Gly Asp Gln Thr Lys Ala Val Ile Glu Asp Ile Leu Glu Tyr Gly Gln
       210                 215                 220
   Cys Ser Lys Val Gln Asp Val Leu Cys Asp Asp Arg Tyr Gln Thr Ile
   225                 230                 235                 240
   Lys Leu Phe Thr Ser Val Phe Gly Val Gly Leu Arg Thr Ala Glu Lys
                   245                 250                 255
   Trp Tyr Arg Lys Gly Phe His Ser Leu Glu Glu Val Gln Ala Asp Asn
               260                 265                 270
   Ala Ile His Phe Thr Lys Met Gln Lys Ala Gly Phe Leu Tyr Tyr Asp
           275                 280                 285
   Asp Ile Ser Ala Ala Val Cys Lys Ala Glu Ala Gln Ala Ile Gly Gln
       290                 295                 300
   Ile Val Glu Glu Thr Val Arg Leu Ile Ala Pro Asp Ala Ile Val Thr
   305                 310                 315                 320
   Leu Thr Gly Gly Phe Arg Arg Gly Lys Glu Cys Gly His Asp Val Asp
                   325                 330                 335
   Phe Leu Ile Thr Thr Pro Glu Met Gly Lys Glu Val Trp Leu Leu Asn
               340                 345                 350
   Arg Leu Ile Asn Arg Leu Gln Asn Gln Gly Ile Leu Leu Tyr Tyr Asp
           355                 360                 365
   Ile Val Glu Ser Thr Phe Asp Lys Thr Arg Leu Pro Cys Arg Lys Phe
       370                 375                 380
   Glu Ala Met Asp His Phe Gln Lys Cys Phe Ala Ile Ile Lys Leu Lys
   385                 390                 395                 400
   Lys Glu Leu Ala Ala Gly Arg Val Gln Lys Asp Trp Lys Ala Ile Arg
                   405                 410                 415
   Val Asp Phe Val Ala Pro Pro Val Asp Asn Phe Ala Phe Ala Leu Leu
               420                 425                 430
   Gly Trp Thr Gly Ser Arg Gln Phe Glu Arg Asp Leu Arg Arg Phe Ala
           435                 440                 445
   Arg His Glu Arg Lys Met Leu Leu Asp Asn His Ala Leu Tyr Asp Lys
       450                 455                 460
   Thr Lys Lys Ile Phe Leu Pro Ala Lys Thr Glu Glu Asp Ile Phe Ala
   465                 470                 475                 480
   His Leu Gly Leu Asp Tyr Ile Asp Pro Trp Gln Arg Asn Ala
                   485                 490

## Description

### FIELD OF THE INVENTION

The invention relates to a method of nucleic acid synthesis comprising the use of 3'-O-azidomethyl blocked nucleotide triphosphates which comprises the step of adding a capping group to any uncleaved 3'-O-azidomethyl groups and to the use of kits comprising said capping groups in a method of nucleic acid synthesis.

### BACKGROUND OF THE INVENTION

Nucleic acid synthesis is vital to modern biotechnology. The rapid pace of development in the biotechnology arena has been made possible by the scientific community's ability to artificially synthesise DNA, RNA and proteins.

Artificial DNA synthesis - a £1 billion and growing market - allows biotechnology and pharmaceutical companies to develop a range of peptide therapeutics, such as insulin for the treatment of diabetes. It allows researchers to characterise cellular proteins to develop new small molecule therapies for the treatment of diseases our aging population faces today, such as heart disease and cancer. It even paves the way forward to creating life, as the Venter Institute demonstrated in 2010 when they placed an artificially synthesised genome into a bacterial cell.

However, current DNA synthesis technology does not meet the demands of the biotechnology industry. While the benefits of DNA synthesis are numerous, an oft-mentioned problem prevents the further growth of the artificial DNA synthesis industry, and thus the biotechnology field. Despite being a mature technology, it is practically impossible to synthesise a DNA strand greater than 200 nucleotides in length, and most DNA synthesis companies only offer up to 120 nucleotides. In comparison, an average protein-coding gene is of the order of 2000 - 3000 nucleotides, and an average eukaryotic genome numbers in the billions of nucleotides. Thus, all major gene synthesis companies today rely on variations of a 'synthesise and stitch' technique, where overlapping 40-60-mer fragments are synthesised and stitched together by PCR (see Young, L. et al. (2004) Nucleic Acid Res. 32, e59). Current methods offered by the gene synthesis industry generally allow up to 3 kb in length for routine production.

The reason DNA cannot be synthesised beyond 120-200 nucleotides at a time is due to the current methodology for generating DNA, which uses synthetic chemistry (i.e., phosphoramidite technology) to couple a nucleotide one at a time to make DNA. As the efficiency of each nucleotide-coupling step is 95.0 - 99.0% efficient, it is mathematically impossible to synthesise DNA longer than 200 nucleotides in acceptable yields. The Venter Institute illustrated this laborious process by spending 4 years and 20 million USD to synthesise the relatively small genome of a bacterium (see Gibson, D. G. et al. (2010) Science 329, 52-56).

Known methods of DNA sequencing use template-dependent DNA polymerases to add 3'-reversibly terminated nucleotides to a growing double-stranded substrate (see, Bentley, D. R. et al. (2008) Nature 456, 53-59, or Palla et al. RSC Adv. 2014,4,49342-49246). In the 'sequencing-by-synthesis' process, each added nucleotide contains a dye, allowing the user to identify the exact sequence of the template strand. Albeit on double-stranded DNA, this technology is able to produce strands of between 500-1000 bps long. However, this technology is not suitable for *de novo* nucleic acid synthesis because of the requirement for an existing nucleic acid strand to act as a template.

There is therefore a need to provide an improved method of nucleic acid synthesis that is able to overcome the problems associated with currently available methods.

### SUMMARY OF THE INVENTION

According to a first aspect of the invention, there is provided a method of nucleic acid synthesis, which comprises the steps of:
(a) providing an initiator sequence;
(b) adding a 3'-O-azidomethyl blocked nucleotide triphosphate to said initiator sequence in the presence of terminal deoxynucleotidyl transferase (TdT) or a functional equivalent or fragment thereof which retains the ability to catalyse the addition of a nucleotide onto the 3'-end of a nucleic acid strand,
(c) removal of TdT;
(d) cleaving the 3'-O-azidomethyl group from the 3'-O-azidomethyl blocked nucleotide triphosphate in the presence of a cleaving agent;
(e) removal of the cleaving agent; and
(f) adding a capping group to any uncleaved 3'-O-azidomethyl groups via a 1,3-dipolar cycloaddition reaction.

According to a second aspect of the invention, there is provided the use of a kit to reduce mutations resulting from 3'-O-azidomethyl groups that are not deprotected during the deprotection step in a method of nucleic acid synthesis, wherein said kit comprises a 3'-O-azidomethyl capped nucleotide triphosphate as defined herein, optionally in combination with one or more components selected from: terminal deoxynucleotidyl transferase (TdT) or a functional equivalent or fragment thereof which retains the ability to catalyse the addition of a nucleotide onto the 3'-end of a nucleic acid strand, an initiator sequence, one or more 3'-blocked nucleotide triphosphates, inorganic pyrophosphatase, such as purified, recombinant inorganic pyrophosphatase from *Saccharomyces cerevisiae,* a cleaving agent, an extension solution, a wash solution and/or a cleaving solution; further optionally together with instructions for use of the kit in accordance with the method as defined herein.

### BRIEF DESCRIPTION OF THE FIGURES

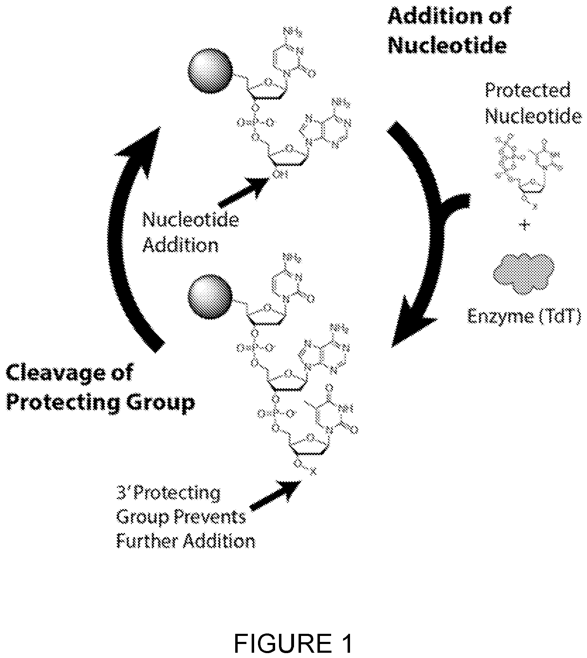

**Figure 1****: Schematic of enzymatic DNA synthesis process.** Starting from the top of the diagram, an immobilised strand of DNA with a deprotected 3'-end is exposed to an extension mixture composed of TdT, a base-specific 3'-blocked nucleotide triphosphate, inorganic pyrophosphatase to reduce the buildup of inorganic pyrophosphate, and appropriate buffers/salts for optimal enzyme activity and stability. The protein adds one protected nucleotide to the immobilised DNA strand (bottom of diagram). The extension mixture is then removed with wash mixture and optionally recycled. The immobilised (n+1) DNA strand is then washed with a cleavage mixture to cleave the 3'-protecting group, enabling reaction in the next cycle. In the cleavage mixture, denaturant may be present to disrupt any secondary structures. During this step, the temperature may be raised to assist in cleavage and disruption of secondary structures. The immobilised DNA is treated with wash mixture to remove leftover cleavage mixture. Steps 1-4 may be repeated with an appropriate nucleotide triphosphate until the desired oligonucleotide sequence is achieved.

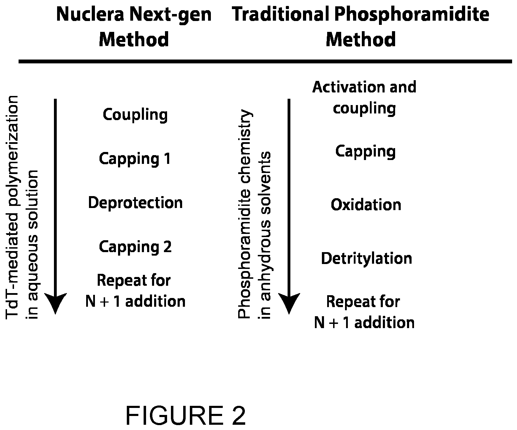

**Figure 2****:** Overview of the capping method of the invention compared with the capping step found in phosphoramidite-based DNA synthesis.

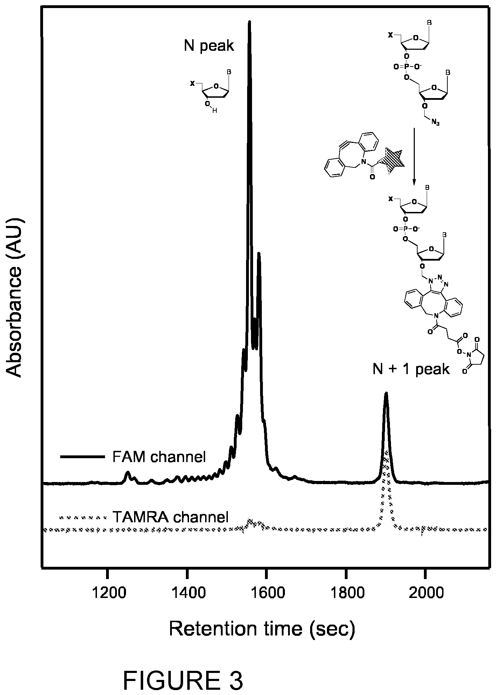

**Figure 3****:** A capillary electrophoresis chromatogram showing a TdT-mediated addition of a 3'-O-azidomethyl thymidine triphosphate to a FAM-labeled DNA initiator. After the 60 min reaction, the products were incubated with DBCO-TAMRA, resulting in the conversion of 3'-O-azidomethyl containing DNA strands into the 1,2,3-triazole adduct. This reaction is evident due to the co-elution of a N + 1 peak with signal in both the FAM and TAMRA channels. The solid star indicates a TAMRA fluorophore. On the molecular structure, B represents any nitrogenous base (such as A, T, C, G, U, hmC, mC, 8-oxo-G, etc.). X represents further nucleotides on the 5'-side.

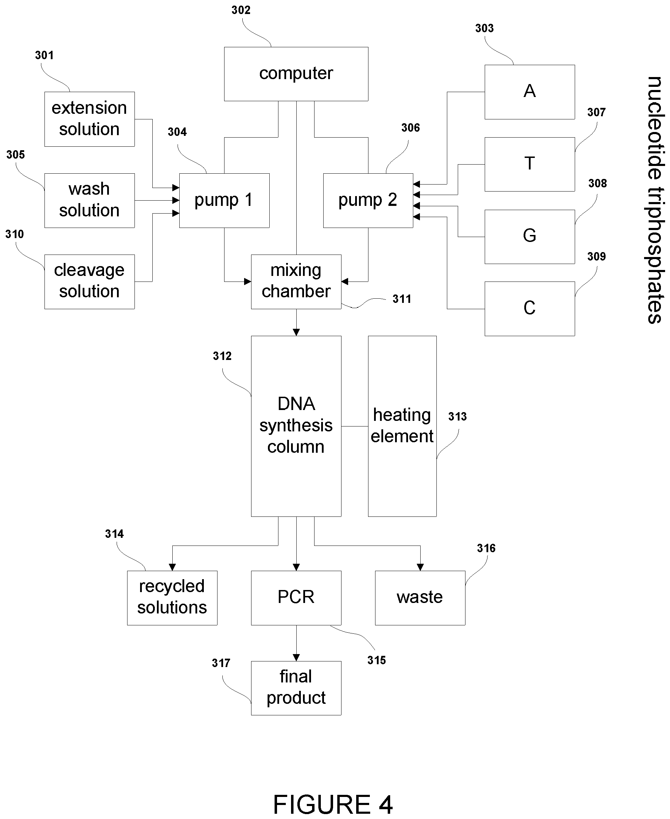

**Figure 4****: Simplified schematic representation of a column-based flow instrument used in DNA synthesis.** A computer (302) controls two pumps and a solution mixing chamber (311). Pump 1 (304) selectively pumps extension solution (301), wash solution (305) or cleavage solution (310) into the mixing chamber. Pump 2 (306) selectively pumps a single 3'-blocked nucleotide triphosphate (TP) solution containing either 3'-blocked A(adenine)TP (303), T(thymine)TP (307), G(guanine)TP (308), or C(cytosine)TP (309) into the chamber. The computer controlled mixing chamber then passes appropriate solution ratios from pump 1 and pump 2 into a column based DNA synthesis chamber (312). A heating element (313) ensures that the DNA synthesis column remains at the necessary temperature for the synthesis process to take place. Upon exiting the DNA synthesis chamber, the reaction solution either enters a recycling vessel (314) for future use, a waste vessel (316) or moves on to a polymerase chain reaction (PCR) step (315) for amplification of the resultant DNA. PCR completion leads to the final product (317).

### DETAILED DESCRIPTION OF THE INVENTION

According to a first aspect of the invention, there is provided a method of nucleic acid synthesis, which comprises the steps of:
(a) providing an initiator sequence;
(b) adding a 3'-O-azidomethyl blocked nucleotide triphosphate to said initiator sequence in the presence of terminal deoxynucleotidyl transferase (TdT) or a functional equivalent or fragment thereof which retains the ability to catalyse the addition of a nucleotide onto the 3'-end of a nucleic acid strand,
(c) removal of TdT;
(d) cleaving the 3'-O-azidomethyl group from the 3'-O-azidomethyl blocked nucleotide triphosphate in the presence of a cleaving agent;
(e) removal of the cleaving agent; and
(f) adding a capping group to any uncleaved 3'-O-azidomethyl groups via a 1,3-dipolar cycloaddition reaction.

The use described herein has significant advantages, such as the ability to rapidly produce long lengths of DNA while still maintaining a high accuracy and yield without using any toxic organic solvents.

The 3'-O-azidomethyl represents one example of a protecting group which may be used to reversibly block nucleotide triphosphates in order to control the nucleic acid sequence during TdT-mediated coupling (see Figure 1). During "deprotection," the nucleotide added to a growing nucleic acid strand is deprotected and readied for the subsequent coupling step. This deprotection step is reported to be quantitative (Guo et al., Proc. Natl. Acad. Sci. 2008), but is not necessarily 100%. The present inventors have identified that a population of unreacted strands may survive through to the subsequent deprotection step, where they are deprotected to reveal the reactive hydroxyl group. This strand as a result becomes an N - 1 mutant.

In addition to the potential to generate N - 1 mutants from the deprotection step, the "coupling" step can also generate N - 1 mutants if TdT fails to add a 3'-O-azidomethyl nucleotide triphosphate to each available free 3'-OH nucleic acid strand. Due to the iterative nature of DNA synthesis, errors are amplified geometrically (e.g., 99% coupling efficiency results in an effective yield of 0.99ⁿ, where n is the quantity of steps). Thus, small errors in each round result in loss of yield and control of nucleic acid sequence over time. Additionally, separating N - 1 or more mutants from the desired sequence represents a significant challenge.

In order to overcome this problem, the invention makes use of 1,3-dipolar cycloaddition click chemistry. The advantages of the invention reduce the likelihood of (1) mutations resulting from unreacted nucleic acid strands during the coupling step (Capping 1) and (2) mutations resulting from 3'-O-azidomethyl groups that are not deprotected during the deprotection step (Capping 2).

Traditional phosphoramidite DNA synthesis (see Figure 2) utilises acetylation chemistry to cap unreacted hydroxyl groups, following the coupling step. Other capping methods involve phosphitylation. However, such chemistry is not suitable for a TdT-mediated synthesis method. Firstly, the commonly used acetylating reagents, acetic anhydride and N-methylimidazole (catalyst), are not stable under aqueous environments. Secondly, the 3'-O-azidomethyl, rather than the deprotected 3'-OH, needs to be blocked to prevent deletion mutants after the deprotection step. Finally, in phosphoramidite chemistry, nucleic acids are synthesised 3' to 5', whereas the method of the invention synthesises nucleic acids 5' to 3'. As a result, a secondary alcohol (3'-OH) rather than a primary alcohol (5'-OH) would be exposed. A secondary alcohol is less reactive than a primary alcohol and thus more difficult to react in a quantitative fashion.

An issue encountered with acetylation capping chemistry is the removal of the acetyl cap during the post-synthesis ammonia cleavage/deprotection step. Removal of the cap regenerates the hydroxyl in failure sequences, rendering them active in biological processes such as enzymatic reactions. The cycloaddition product is stable under a wide range of conditions, and will remain present in failure sequences throughout the synthesis process of the invention.

In the coupling stage, if TdT fails to add a 3'-O-azidomethyl nucleotide triphosphate to a strand, that strand is left with a free 3'-OH. This 3'-OH can be trapped by incubation with TdT in a second coupling stage (called "Capping 1") with a nucleotide triphosphate that does not contain a free 3'-OH function group for continued strand growth in subsequent cycles, such as 2', 3'-dideoxy nucleotide triphosphate analogs. In an alternative embodiment, a 3'-azido or 3'-amino nucleotide triphosphate is added by TdT to an unreacted strand.

In the deprotecting stage, if tris(2-carboxyethyl)phosphine (TCEP) mediated deprotection fails to deprotect a 3'-O-azidomethyl group on a nucleic acid strand, the strand is left with an unreacted 3'-O-azidomethyl group. By reacting DNA strands containing a 3'-O-azidomethyl group with a capping group (called "Capping 2"), the inventors have surprisingly shown that the capping group serves as a suitable moiety to irreversibly cap a strand (see Figure 3).

### Capping Groups

In one embodiment, the capping group is an irreversible capping group.

In one embodiment, the capping group is a dipolarophile. In a further embodiment,
the dipolarophile is an alkyne. In a yet further embodiment, the alkyne is a strained alkyne.

In a yet further embodiment, the dipolarophile is dibenzocyclooctyne-amine (CAS Number: 1255942-06-3).

When the capping group is a dibenzocyclooctyne analogue, step (f) may typically comprise the reaction shown in Scheme 1: 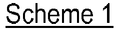 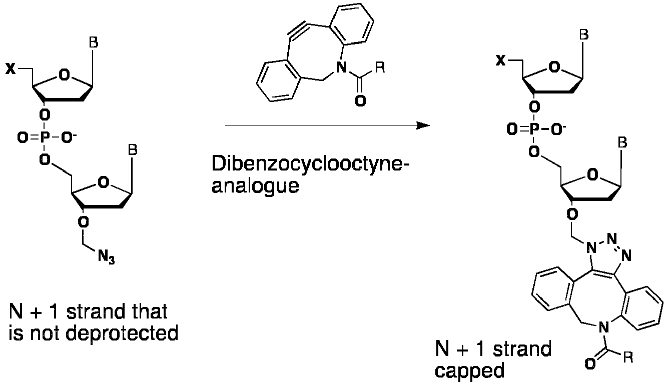 wherein X represents further nucleotides on the 5'-side. B represents any nitrogenous base, such as such as A, T, C, G, U, hmC, mC, 8-oxo-G, etc. R represents any functional group such as fluorophores, biotin, amine, carboxylic acid, maleimide, etc.

In this embodiment of the invention shown in Scheme 1, a dibenzocyclooctyne analogue is reacted with an N + 1 strand that has failed to deprotect. The reaction results in a triazole adduct that is not labile to reducing agents, thereby irreversibly capping the strand.

In one embodiment, the 1,3-dipolar cycloaddition reaction of step (f) comprises an uncatalysed cycloaddition reaction.

In an alternative embodiment, the 1,3-dipolar cycloaddition reaction of step (f) comprises a cycloaddition reaction catalysed by a copper, ruthenium, or other transition metal-based catalyst.

In addition to capping deletion mutants, the capping group may serve as a purification handle to sequester deletion mutants. Thus, in one embodiment, the capping group comprises one half of a binding pair, such as biotin. Such coupling would allow capture of deletion mutants by exposure to avidin or streptavidin.

The capping group may also serve as a handle to facilitate liquid chromatographic separation of the product from deletion mutants. Such handles include fluorous tags (e.g.:CₙF₂ₙ₊₁) for use in fluorous HPLC. Thus, in one embodiment, the capping group comprises a fluorine-containing moiety.

In order to quantify the amount of deletion mutants generated over n cycles, a fluorescently-tagged capping group can be used. Thus, in one embodiment, the capping group comprises a fluorescent moiety. Such tagging allows for direct quantification via fluorescent spectroscopy to obtain the quantity of deletion mutants produced in the present DNA synthesis method as a result of deprotection failure.

In one embodiment, the 3'-O-azidomethyl blocked nucleotide triphosphate is selected from a compound of formula (I), (II), (III) or (IV): 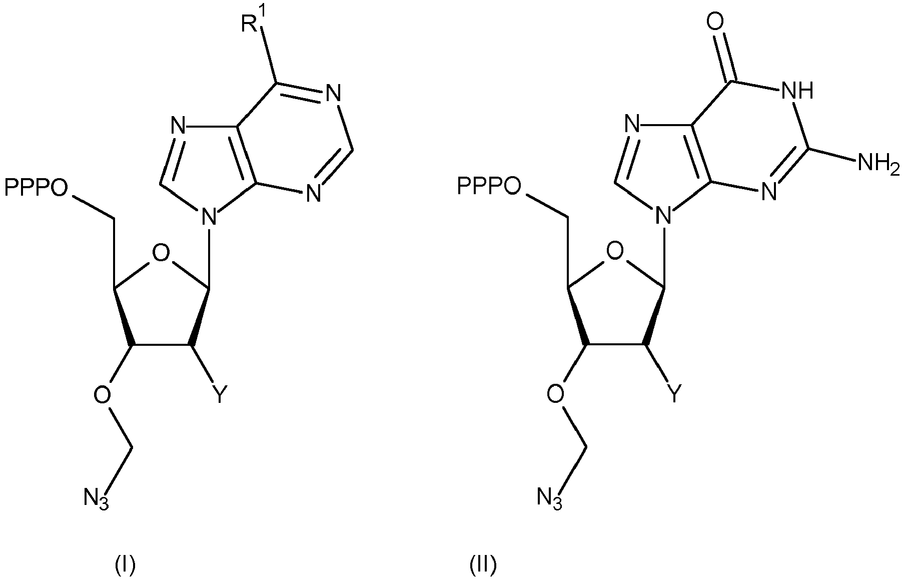 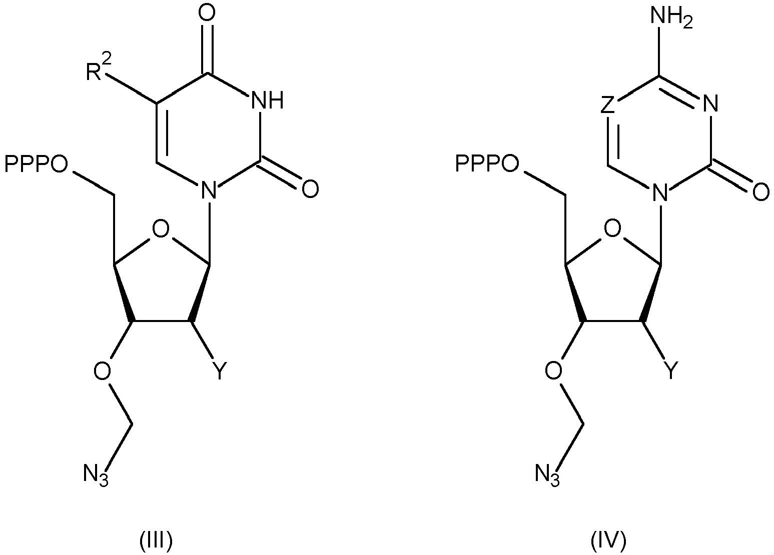 wherein
R¹ represents NR^{a}R^{b}, wherein R^{a} and R^{b} independently represent hydrogen or C₁₋₆ alkyl;
R² represents hydrogen, C₁₋₆ alkoxy, COH, COOH or C₁₋₆ alkyl optionally substituted by one or more OH or COOH groups;
Y represents hydrogen, hydroxyl or halogen; and
Z represents CR⁴ or N, wherein R⁴ represents hydrogen, C₁₋₆ alkoxy, COH, COOH or C₁₋₆ alkyl optionally substituted by one or more OH or COOH groups.

In one embodiment which may be mentioned, there is provided a compound of formula (I), (II), (III) or (IV) wherein
R¹ represents NR^{a}R^{b}, wherein R^{a} and R^{b} independently represent hydrogen or C₁₋₆ alkyl;
R² represents hydrogen, C₁₋₆ alkoxy, COH, COOH or C₁₋₆ alkyl optionally substituted by one or more OH or COOH groups;
Y represents hydrogen or hydroxyl; and
Z represents CR⁴ or N, wherein R⁴ represents C₁₋₆ alkoxy, COH, COOH or C₁₋₆ alkyl optionally substituted by one or more OH or COOH groups.

It will be understood that 'PPP' in the structures shown herein represents a triphosphate group.

References to the term 'C₁₋₆ alkyl' as used herein as a group or part of a group refers to a linear or branched saturated hydrocarbon group containing from 1 to 6 carbon atoms. Examples of such groups include methyl, ethyl, butyl, n-propyl, isopropyl and the like.

References to the term 'C₁₋₆ alkoxy' as used herein refer to an alkyl group bonded to oxygen via a single bond (*i.e.* R-O). Such references include those with straight and branched alkyl chains containing 1 to 6 carbon atoms, such as methoxy (or methyloxy), ethyloxy, n-propyloxy, iso-propyloxy, n-butyloxy and 2-methylpropyloxy.

References to the term 'COOH' or 'CO₂H' refer to a carboxyl group (or carboxy) which consists of a carbonyl (C=O) and a hydroxyl (O-H) group. References to the term 'COH' refer to a formyl group which consists of a carbonyl (C=O) group bonded to hydrogen.

The term 'N₃' (drawn structurally as -N=N⁺=N⁻) refers to an azido group.

In one embodiment, R^{a} and R^{b} both represent hydrogen (*i.e.* R¹ represents NH₂).

In an alternative embodiment, R^{a} represents hydrogen and R^{b} represents methyl (*i.e.* R¹ represents NHCH₃).

In one embodiment, R² represents hydrogen, methyl or methoxy. In a further embodiment, R² represents hydrogen. In an alternative embodiment, R² represents methyl. In a yet further alternative embodiment, R² represents methoxy.

In one embodiment, Y represents hydrogen.

In an alternative embodiment, Y represents hydroxyl.

In one embodiment, Z represents N.

In an alternative embodiment, Z represents CR⁴.

In one embodiment, R⁴ represents methoxy, COOH or COH. In a further embodiment, R⁴ represents methoxy. In an alternative embodiment, R⁴ represents COOH. In a yet further alternative embodiment, R⁴ represents COH.

In one embodiment, the 3'-blocked nucleotide triphosphate is selected from:

| **Structure** | **Name** | **Example number** |
|---|---|---|
| 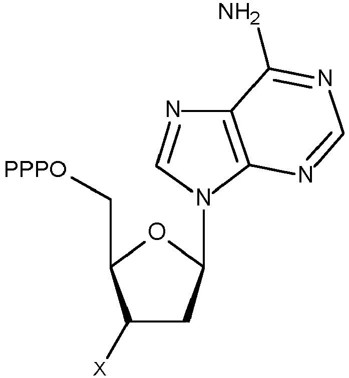 | Deoxyadenosine triphosphate | E1 |
| 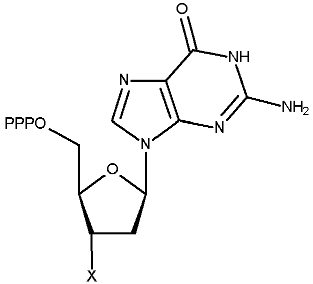 | Deoxyguanosine triphosphate | E2 |
| 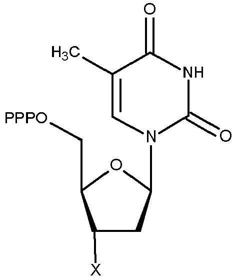 | Deoxythymidine triphosphate | E3 |
| 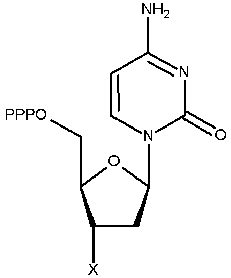 | Deoxycytidine triphosphate | E4 |
| 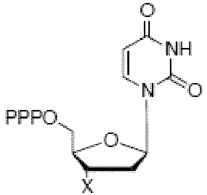 | 2'-deoxy-uridine triphosphate | E5 |
| 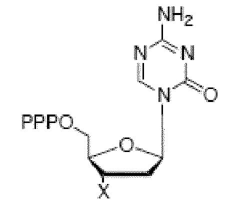 | 5-aza-2'-deoxy-cytidine-triphosphate | E6 |
| 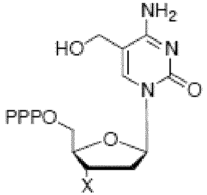 | 5-hydroxymethyl-deoxycytidine triphosphate | E7 |
| 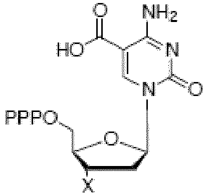 | 5-carboxy-deoxycytidine triphosphate | E8 |
| 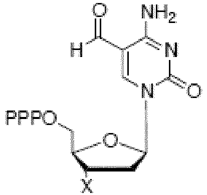 | 5-formyl-deoxycytidine triphosphate | E9 |
| 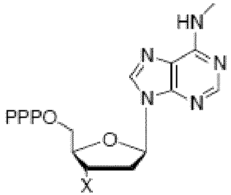 | N6-methyladenosine triphosphate | E10 |
| 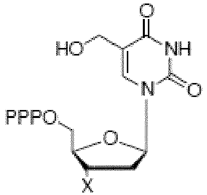 | 5-hydroxymethyl-deoxy-uridine triphosphate | E11 |

wherein 'X' represents -O-CH₂-N₃.

According to a further aspect of the disclosure, there is provided a capped nucleotide triphosphate selected from a compound of formula (I)^{a}, (II)^{a}, (III)^{a} or (IV)^{a}: 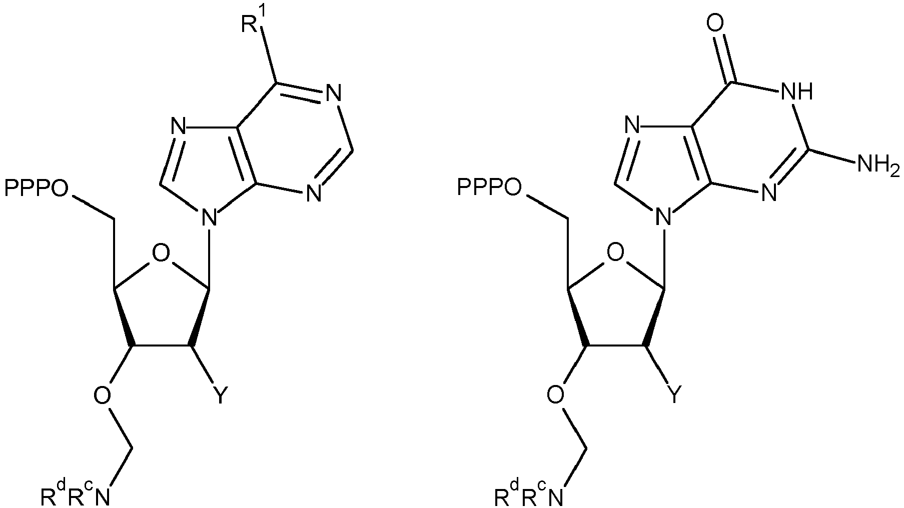 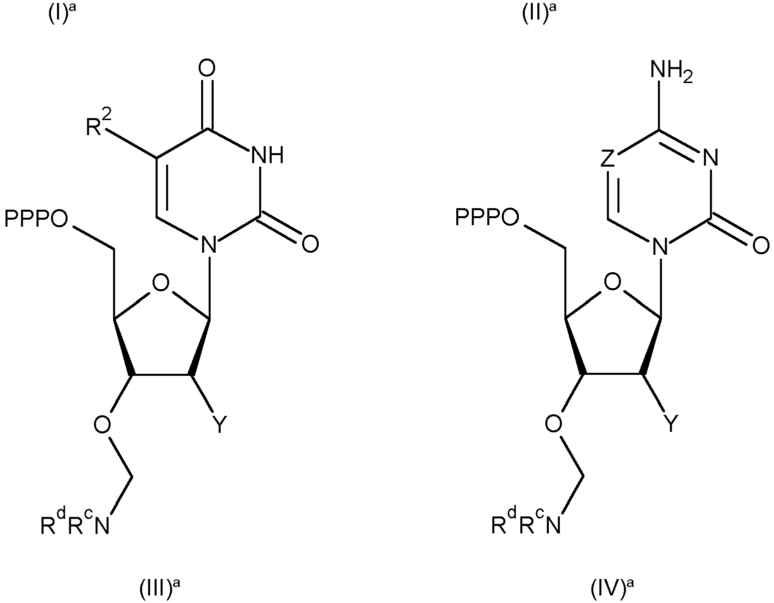 wherein
R¹ represents NR^{a}R^{b}, wherein R^{a} and R^{b} independently represent hydrogen or C₁₋₆ alkyl;
R² represents hydrogen, C₁₋₆ alkoxy, COH, COOH or C₁₋₆ alkyl optionally substituted by one or more OH or COOH groups;
Y represents hydrogen, hydroxyl or halogen;
Z represents CR⁴ or N, wherein R⁴ represents hydrogen, C₁₋₆ alkoxy, COH, COOH or C₁₋₆ alkyl optionally substituted by one or more OH or COOH groups; and
R^{c} and R^{d} together with the nitrogen atom to which they are attached join to form a triazole ring fused to one or more carbocyclic or heterocyclic ring systems, wherein said ring systems may be optionally substituted by any suitable functional groups, such as an amine, carboxylic acid, maleimide, one half of a binding pair (e.g. biotin), a fluorine containing moiety or a fluorescent moiety.

There is also disclosed herein a compound of formula (I)^{a}, (II)^{a}, (III)^{a} or (IV)^{a} wherein
R¹ represents NR^{a}R^{b}, wherein R^{a} and R^{b} independently represent hydrogen or C₁₋₆ alkyl;
R² represents hydrogen, C₁₋₆ alkoxy, COH, COOH or C₁₋₆ alkyl optionally substituted by one or more OH or COOH groups;
Y represents hydrogen or hydroxyl;
Z represents CR⁴ or N, wherein R⁴ represents C₁₋₆ alkoxy, COH, COOH or C₁₋₆ alkyl optionally substituted by one or more OH or COOH groups; and
R^{c} and R^{d} together with the nitrogen atom to which they are attached join to form a triazole ring fused to one or more carbocyclic or heterocyclic ring systems, wherein said ring systems may be optionally substituted by any suitable functional groups, such as an amine, carboxylic acid, maleimide, one half of a binding pair (e.g. biotin), a fluorine containing moiety or a fluorescent moiety.

-NR^{c}R^{d} may represent a group of formula (V): 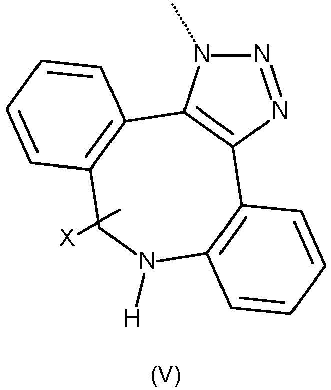 wherein X represents one or more suitable functional groups, such as an amine, carboxylic acid, maleimide, one half of a binding pair (e.g. biotin), a fluorine containing moiety or a fluorescent moiety.

According to a further aspect of the disclosure, there is provided the use of an alkyne containing reagent as a 3'-O-azidomethyl capping group.

The alkyne containing reagent may be selected from a compound of formula (VI): 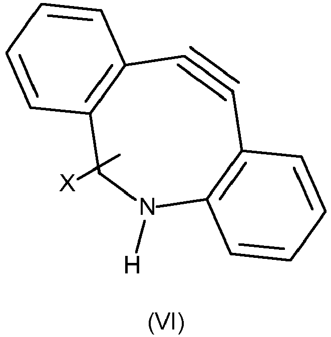 wherein X represents one or more suitable functional groups, such as an amine, carboxylic acid, maleimide, one half of a binding pair (e.g. biotin), a fluorine containing moiety or a fluorescent moiety. When the alkyne containing reagent comprises a compound of formula (VI) the process of capping a 3'-O-azidomethyl group typically comprises a cycloaddition reaction as described hereinbefore in Scheme 1.

Alternatively, the alkyne containing reagent may be selected from a compound of formula (VII): 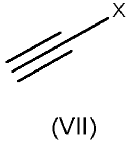 wherein X is as defined hereinbefore. When the alkyne containing reagent comprises a compound of formula (VII) the process of capping a 3'-O-azidomethyl group typically comprises a cycloaddition reaction in accordance with procedures known to the skilled person, such as a cycloaddition reaction catalysed by a transition metal-based catalyst.

According to a further aspect of the disclosure, there is provided a 3'-O-azidomethyl capping group selected from a compound of formula (VI) or (VII).

### Terminal deoxynucleotidyl transferase (TdT) enzymes

References herein to terminal deoxynucleotidyl transferase (TdT) enzyme include references to purified and recombinant forms of said enzyme. It will be appreciated that references herein to "homology" are to be understood as meaning the similarity between two protein sequences, e.g.: SEQ ID NO: X and SEQ ID NO: Y, which is calculated by addition of the the common amino acids between aligned sequences SEQ ID NO: X and SEQ ID NO: Y, divided by the longer length of either SEQ ID NO: X or SEQ ID NO: Y, expressed as a percentage.

In one embodiment, the terminal deoxynucleotidyl transferase (TdT) is a natural TdT or non-natural TdT or a functional equivalent or fragment thereof which retains the ability to catalyse the addition of a nucleotide onto the 3'-end of a nucleic acid strand.

It will be understood that the term 'functional equivalent' refers to the polypeptides which are different to the exact sequence of a TdT (such as *Bos taurus* TdT), but can perform the same function, *i.e.* catalyse the addition of a nucleotide triphosphate onto the 3'-end of a DNA strand in a template dependent manner.

In one embodiment, the terminal deoxynucleotidyl transferase (TdT) enzyme comprises an amino acid sequence selected from any one of SEQ ID NOS: 1 to 5 and 8 or a functional equivalent or fragment thereof which retains the ability to catalyse the addition of a nucleotide onto the 3'-end of a nucleic acid strand having at least 20% sequence homology to said amino acid sequence.

In a further embodiment, the terminal deoxynucleotidyl transferase (TdT) enzyme comprises an amino acid sequence selected from SEQ ID NO: 1. The amino acid sequence of SEQ ID NO.1 is the terminal deoxynucleotidyl transferase (TdT) sequence from *Sarcophilus harrisii* (UniProt: G3VQ55). *Sarcophilus harrisii* (also known as the Tasmanian devil) is a carnivorous marsupial of the family Dasyuridae, now found in the wild only on the Australian island state of Tasmania.

In a further embodiment, the terminal deoxynucleotidyl transferase (TdT) enzyme comprises an amino acid sequence selected from SEQ ID NO: 2. The amino acid sequence of SEQ ID NO.2 is the terminal deoxynucleotidyl transferase (TdT) sequence from *Lepisosteus oculatus* (UniProt: W5MK82). *Lepisosteus oculatus* (also known as the spotted gar) is a primitive freshwater fish of the family Lepisosteidae, native to North America from the Lake Erie and southern Lake Michigan drainages south through the Mississippi River basin to Gulf Slope drainages, from lower Apalachicola River in Florida to Nueces River in Texas, USA.

In a further embodiment, the terminal deoxynucleotidyl transferase (TdT) enzyme comprises an amino acid sequence selected from SEQ ID NO: 3. The amino acid sequence of SEQ ID NO.3 is the terminal deoxynucleotidyl transferase (TdT) sequence from *Chinchilla lanigera* (NCBI Reference Sequence: XP_005407631.1; http://www.ncbi.nlm.nih.gov/protein/533189443). *Chinchilla lanigera* (also known as the long-divided by the longer length of either SEQ ID NO: X or SEQ ID NO: Y, expressed as a percentage.

In one embodiment, the terminal deoxynucleotidyl transferase (TdT) is a natural TdT or non-natural TdT or a functional equivalent or fragment thereof.

It will be understood that the term 'functional equivalent' refers to the polypeptides which are different to the exact sequence of a TdT (such as *Bos taurus* TdT), but can perform the same function, *i.e.* catalyse the addition of a nucleotide triphosphate onto the 3'-end of a DNA strand in a template dependent manner.

In one embodiment, the terminal deoxynucleotidyl transferase (TdT) enzyme comprises an amino acid sequence selected from any one of SEQ ID NOS: 1 to 5 and 8 or a functional equivalent or fragment thereof having at least 20% sequence homology to said amino acid sequence.

In a further embodiment, the terminal deoxynucleotidyl transferase (TdT) enzyme comprises an amino acid sequence selected from SEQ ID NO: 1. The amino acid sequence of SEQ ID NO.1 is the terminal deoxynucleotidyl transferase (TdT) sequence from *Sarcophilus harrisii* (UniProt: G3VQ55). *Sarcophilus harrisii* (also known as the Tasmanian devil) is a carnivorous marsupial of the family Dasyuridae, now found in the wild only on the Australian island state of Tasmania.

In a further embodiment, the terminal deoxynucleotidyl transferase (TdT) enzyme comprises an amino acid sequence selected from SEQ ID NO: 2. The amino acid sequence of SEQ ID NO.2 is the terminal deoxynucleotidyl transferase (TdT) sequence from *Lepisosteus oculatus* (UniProt: W5MK82). *Lepisosteus oculatus* (also known as the spotted gar) is a primitive freshwater fish of the family Lepisosteidae, native to North America from the Lake Erie and southern Lake Michigan drainages south through the Mississippi River basin to Gulf Slope drainages, from lower Apalachicola River in Florida to Nueces River in Texas, USA.

In a further embodiment, the terminal deoxynucleotidyl transferase (TdT) enzyme comprises an amino acid sequence selected from SEQ ID NO: 3. The amino acid sequence of SEQ ID NO.3 is the terminal deoxynucleotidyl transferase (TdT) sequence from *Chinchilla lanigera* (NCBI Reference Sequence: XP_005407631.1; http://www.ncbi.nlm.nih.gov/protein/533189443). *Chinchilla lanigera* (also known as the long-tailed chinchilla, Chilean, coastal, common chinchilla, or lesser chinchilla), is one of two species of rodents from the genus *Chinchilla,* the other species being *Chinchilla chinchilla.*

In a further embodiment, the terminal deoxynucleotidyl transferase (TdT) enzyme comprises an amino acid sequence selected from SEQ ID NO: 4. The amino acid sequence of SEQ ID NO.4 is the terminal deoxynucleotidyl transferase (TdT) sequence from *Otolemur garnettii* (UniProt: A4PCE6). *Otolemur garnettii* (also known as the northern greater galago, Garnett's greater galago or small-eared greater galago), is a nocturnal, arboreal primate endemic to Africa.

In a further embodiment, the terminal deoxynucleotidyl transferase (TdT) enzyme comprises an amino acid sequence selected from SEQ ID NO: 5. The amino acid sequence of SEQ ID NO.5 is the terminal deoxynucleotidyl transferase (TdT) sequence from *Sus scrofa* (UniProt: F1SBG2). *Sus scrofa* (also known as the wild boar, wild swine or Eurasian wild pig) is a suid native to much of Eurasia, North Africa and the Greater Sunda Islands.

In an alternative embodiment, the terminal deoxynucleotidyl transferase (TdT) enzyme comprises an amino acid sequence selected from *Bos taurus* (UniProt: P06526). *Bos taurus (also known* as cattle, or colloquially cows) are the most common type of large domesticated ungulates. They are a prominent modern member of the subfamily Bovinae, are the most widespread species of the genus Bos.

In a further embodiment, the terminal deoxynucleotidyl transferase (TdT) enzyme comprises an amino acid sequence selected from SEQ ID NO: 8. The amino acid sequence of SEQ ID NO: 8 is a variant of SEQ ID NO: 2 which has been engineered for improved activity by alteration of the amino acid sequence.

In a further embodiment, the terminal deoxynucleotidyl transferase (TdT) enzyme comprises an amino acid sequence selected from SEQ ID NOS: 1, 2 or 8.

In an alternative embodiment, the terminal deoxynucleotidyl transferase (TdT) enzyme comprises an amino acid sequence selected from a modified derivative of SEQ ID NO: 6 (i.e. a non-natural, mutated derivative of SEQ ID NO: 6). The amino acid sequence of SEQ ID NO: 6 is the terminal deoxynucleotidyl transferase (TdT) sequence from *Bos taurus* (UniProt: P06526). *Bos taurus* (also known as cattle, or colloquially cows) are the most common type of large domesticated ungulates. They are a prominent modern member of the subfamily Bovinae, are the most widespread species of the genus Bos.

References herein to 'fragment' include, for example, functional fragments with a C-terminal truncation, or with an N-terminal truncation. Fragments are suitably greater than 10 amino acids in length, for example greater than 15, 20, 25, 30, 35, 40, 45, 50, 55, 60, 65, 70, 75, 80, 85, 90, 95, 100, 110, 120, 130, 140, 150, 160, 170, 180, 190, 200, 210, 220, 230, 240, 250, 260, 270, 280, 290, 300, 310, 320, 330, 340, 350, 360, 370, 380, 390, 400, 410, 420, 430, 440, 450, 460, 470, 480, 490 or 500 amino acids in length.

In one embodiment, the terminal deoxynucleotidyl transferase (TdT) has at least 25% homology with the TdTs described herein, such as at least 30%, 40%, 50%, 55%, 60%, 65%, 70%, 75%, 80%, 85%, 90%, 95%, 96%, 97%, 98% or 99% homology.

### Nucleic Acid Synthesis

References herein to a "method of nucleic acid synthesis" include methods of synthesising lengths of DNA (deoxyribonucleic acid) or RNA (ribonucleic acid) wherein a strand of nucleic acid (n) is extended by adding a further nucleotide (n+1). In one embodiment, the nucleic acid is DNA. In an alternative embodiment, the nucleic acid is RNA.

References herein to "method of DNA synthesis" refer to a method of DNA strand synthesis wherein a DNA strand (n) is extended by adding a further nucleotide (n+1). The method described herein provides a novel use of the terminal deoxynucleotidyl transferases of the invention and 3'-blocked nucleotide triphosphates to sequentially add nucleotides in *de novo* DNA strand synthesis which has several advantages over the DNA synthesis methods currently known in the art.

It will be understood that steps (b) to (f) of the method may be repeated multiple times to produce a DNA or RNA strand of a desired length. Therefore, in one embodiment, greater than 1 nucleotide is added to the initiator sequence, such as greater than 5, 10, 20, 30, 40, 50, 60, 70, 80, 90, 100, 110 or 120 nucleotides are added to the initiator sequence by repeating steps (b) to (f). In a further embodiment, greater than 200 nucleotides are added, such as greater than 300, 400, 500, 600, 700, 800, 900, 1000, 1250, 1500, 1750, 2000, 2250, 2500, 2750, 3000, 4000, 5000, 6000, 7000, 8000, 9000 or 10000 nucleotides.

### 3'-blocked nucleotide triphosphates

References herein to 'nucleotide triphosphates' refer to a molecule containing a nucleoside (*i.e.* a base attached to a deoxyribose or ribose sugar molecule) bound to three phosphate groups. Examples of nucleotide triphosphates that contain deoxyribose are: deoxyadenosine triphosphate (dATP), deoxyguanosine triphosphate (dGTP), deoxycytidine triphosphate (dCTP) or deoxythymidine triphosphate (dTTP). Examples of nucleotide triphosphates that contain ribose are: adenosine triphosphate (ATP), guanosine triphosphate (GTP), cytidine triphosphate (CTP) or uridine triphosphate (UTP). Other types of nucleosides may be bound to three phosphates to form nucleotide triphosphates, such as artificial nucleosides.

Therefore, references herein to '3'-O-azidomethyl blocked nucleotide triphosphates' refer to nucleotide triphosphates (*e.g*., dATP, dGTP, dCTP or dTTP) which have an additional azidomethyl group (i.e. -O-CH₂-N₃) on the 3' end which prevents further addition of nucleotides, *i.e.,* by replacing the 3'-OH group with a 3'-O-azidomethyl protecting group.

It will be understood that references herein to '3'-block', '3'-blocking group' or '3'-protecting group' refer to the group attached to the 3' end of the nucleotide triphosphate which prevents further nucleotide addition. The present method uses reversible 3'-protecting groups (i.e. 3'-O-azidomethyl) which can be removed by cleavage to allow the addition of further nucleotides.

### Cleaving agent

References herein to 'cleaving agent' refer to a substance which is able to cleave the 3'-O-azidomethyl blocking group from the 3'-blocked nucleotide triphosphate.

The 3'-O-azidomethyl blocking group may be quantitatively removed in aqueous solution with documented compounds which may be used as cleaving agents (for example, see: Wuts, P.G.M. & Greene, T.W. (2012) 4th Ed., John Wiley & Sons; Hutter, D. et al. (2010) Nucleosides Nucleotides Nucleic Acids 29, 879-895; EP 1560838 and US 7,795,424).

In one embodiment, the cleaving agent is a chemical cleaving agent. In an alternative embodiment, the cleaving agent is an enzymatic cleaving agent.

In one embodiment, tris(2-carboxyethyl)phosphine (TCEP) can be used to cleave a 3'-O-azidomethyl group.

In one embodiment, the cleaving agent is added in the presence of a cleavage solution comprising a denaturant, such as urea, guanidinium chloride, formamide or betaine. The addition of a denaturant has the advantage of being able to disrupt any undesirable secondary structures in the DNA. In a further embodiment, the cleavage solution comprises one or more buffers. It will be understood by the person skilled in the art that the choice of buffer is dependent on the exact cleavage chemistry and cleaving agent required.

### Initiator Sequences

References herein to an 'initiator sequence' refer to a short oligonucleotide with a free 3'-end which the 3'-O-azidomethyl blocked nucleotide triphosphate can be attached to. In one embodiment, the initiator sequence is a DNA initiator sequence. In an alternative embodiment, the initiator sequence is an RNA initiator sequence.

References herein to a 'DNA initiator sequence' refer to a small sequence of DNA which the 3'-blocked nucleotide triphosphate can be attached to, *i.e.* DNA will be synthesised from the end of the DNA initiator sequence.

In one embodiment, the initiator sequence is between 5 and 50 nucleotides long, such as between 5 and 30 nucleotides long (i.e. between 10 and 30), in particular between 5 and 20 nucleotides long (i.e., approximately 20 nucleotides long), more particularly 5 to 15 nucleotides long, for example 10 to 15 nucleotides long, especially 12 nucleotides long.

In one embodiment, the initiator sequence has the following sequence: 5'-CGTTAACATATT-3' (SEQ ID NO: 7).

In one embodiment, the initiator sequence is single-stranded. In an alternative embodiment, the initiator sequence is double-stranded. It will be understood by persons skilled in the art that a 3'-overhang (*i.e.,* a free 3'-end) allows for efficient addition.

In one embodiment, the initiator sequence is immobilised on a solid support. This allows TdT and the cleaving agent to be removed (in steps (c) and (e), respectively) without washing away the synthesised nucleic acid. The initiator sequence may be attached to a solid support stable under aqueous conditions so that the method can be easily performed via a flow setup or microarray setup.

In one embodiment, the initiator sequence is immobilised on a solid support via a reversible interacting moiety, such as a chemically-cleavable linker, an antibody/immunogenic epitope, a biotin/biotin binding protein (such as avidin or streptavidin), or glutathione-GST tag. Therefore, in a further embodiment, the method additionally comprises extracting the resultant nucleic acid by removing the reversible interacting moiety in the initiator sequence, such as by incubating with proteinase K.

In a further embodiment, the initiator sequence is immobilised on a solid support via a chemically-cleavable linker, such as a disulfide, allyl, or azide-masked hemiaminal ether linker. Therefore, in one embodiment, the method additionally comprises extracting the resultant nucleic acid by cleaving the chemical linker through the addition of tris(2-carboxyethyl)phosphine (TCEP) or dithiothreitol (DTT) for a disulfide linker; palladium complexes for an allyl linker; or TCEP for an azide-masked hemiaminal ether linker.

In one embodiment, the resultant nucleic acid is extracted and amplified by polymerase chain reaction using the nucleic acid bound to the solid support as a template. The initiator sequence could therefore contain an appropriate forward primer sequence and an appropriate reverse primer could be synthesised.

In an alternative embodiment, the immobilised initiator sequence contains at least one restriction site. Therefore, in a further embodiment, the method additionally comprises extracting the resultant nucleic acid by using a restriction enzyme.

The use of restriction enzymes and restriction sites to cut nucleic acids in a specific location is well known in the art. The choice of restriction site and enzyme can depend on the desired properties, for example whether 'blunt' or 'sticky' ends are required. Examples of restriction enzymes include: Alul, BamHI, EcoRI, EcoRII, EcoRV, Haell, Hgal, Hindlll, Hinfl, NotI, PstI, Pvull, Sall, Sau3A, ScaI, SmaI, TaqI and XbaI.

### Nucleic acid synthesis method

In one embodiment, the terminal deoxynucleotidyl transferase (TdT) of the invention is added in the presence of an extension solution comprising one or more buffers (e.g., Tris or cacodylate), one or more salts (e.g., Na⁺, K⁺, Mg²⁺, Mn²⁺, Cu²⁺, Zn²⁺, Co²⁺, etc., all with appropriate counterions, such as Cl⁻) and inorganic pyrophosphatase (e.g., the *Saccharomyces cerevisiae* homolog). It will be understood that the choice of buffers and salts depends on the optimal enzyme activity and stability.

The use of an inorganic pyrophosphatase helps to reduce the build-up of pyrophosphate due to nucleotide triphosphate hydrolysis by TdT. Therefore, the use of an inorganic pyrophosphatase has the advantage of reducing the rate of (1) backwards reaction and (2) TdT strand dismutation. Thus, according to a further aspect of the invention, there is provided the use of inorganic pyrophosphatase in a method of nucleic acid synthesis. In one embodiment, the inorganic pyrophosphatase comprises purified, recombinant inorganic pyrophosphatase from *Saccharomyces cerevisiae.*

In one embodiment, step (b) is performed at a pH range between 5 and 10. Therefore, it will be understood that any buffer with a buffering range of pH 5-10 could be used, for example cacodylate, Tris, HEPES or Tricine, in particular cacodylate or Tris.

In one embodiment, step (d) is performed at a temperature less than 99°C, such as less than 95°C, 90°C, 85°C, 80°C, 75°C, 70°C, 65°C, 60°C, 55°C, 50°C, 45°C, 40°C, 35°C, or 30°C. It will be understood that the optimal temperature will depend on the cleavage agent utilised. The temperature used helps to assist cleavage and disrupt any secondary structures formed during nucleotide addition.

In one embodiment, steps (c) and (e) are performed by applying a wash solution. In one embodiment, the wash solution comprises the same buffers and salts as used in the extension solution described herein. This has the advantage of allowing the wash solution to be collected after step (c) and recycled as extension solution in step (b) when the method steps are repeated.

In one embodiment, the method is performed within a flow instrument as shown in **Figure 4****,** such as a microfluidic or column-based flow instrument. The method described herein can easily be performed in a flow setup which makes the method simple to use. It will be understood that examples of commercially available DNA synthesisers (e.g., MerMade 192E from BioAutomation or H-8 SE from K&A) may be optimised for the required reaction conditions and used to perform the method described herein.

In one embodiment, the method is performed on a plate or microarray setup. For example, nucleotides may be individually addressed through a series of microdispensing nozzles using any applicable jetting technology, including piezo and thermal jets. This highly parallel process may be used to generate hybridization microarrays and is also amenable to DNA fragment assembly through standard molecular biology techniques.

In one embodiment, the method additionally comprises amplifying the resultant nucleic acid. Methods of DNA/RNA amplification are well known in the art. For example, in a further embodiment, the amplification is performed by polymerase chain reaction (PCR). This step has the advantage of being able to extract and amplify the resultant nucleic acid all in one step.

The template independent nucleic acid synthesis method described herein has the capability to add a nucleic acid sequence of defined composition and length to an initiator sequence. Therefore, it will be understood by persons skilled in the art, that the method described herein may be used as a novel way to introduce adapter sequences to a nucleic acid library.

If the initiator sequence is not one defined sequence, but instead a library of nucleic acid fragments (for example generated by sonication of genomic DNA, or for example messenger RNA) then this method is capable of *de novo* synthesis of 'adapter sequences' on every fragment. The installation of adapter sequences is an integral part of library preparation for next-generation library nucleic acid sequencing methods, as they contain sequence information allowing hybridisation to a flow cell/solid support and hybridisation of a sequencing primer.

Currently used methods include single stranded ligation, however this technique is limited because ligation efficiency decreases strongly with increasing fragment length. Consequently, current methods are unable to attach sequences longer than 100 nucleotides in length. Therefore, the method described herein allows for library preparation in an alternative fashion to that which is currently possible.

Therefore, in one embodiment, an adapter sequence is added to the initiator sequence. In a further embodiment, the initiator sequence may be a nucleic acid from a library.

### Kits

According to a further aspect of the invention, there is provided the use of a kit to reduce mutations resulting from 3'-O-azidomethyl groups that are not deprotected during the deprotection step in a method of nucleic acid synthesis, wherein said kit comprises a 3'-O-azidomethyl capped nucleotide triphosphate as defined herein, optionally in combination with one or more components selected from: terminal deoxynucleotidyl transferase (TdT) or a functional equivalent or fragment thereof which retains the ability to catalyse the addition of a nucleotide onto the 3'-end of a nucleic acid strand, an initiator sequence, one or more 3'-blocked nucleotide triphosphates, inorganic pyrophosphatase, such as purified, recombinant inorganic pyrophosphatase from *Saccharomyces cerevisiae,* a cleaving agent, an extension solution, a wash solution and/or a cleaving solution; further optionally together with instructions for use of the kit in accordance with the method as defined herein.

The following studies and protocols illustrate embodiments of the methods described herein:
A single-stranded DNA initiator labeled with a 5'-FAM tag was incubated with (1) 15 U *Bos taurus* TdT, (2) required salts (50 mM potassium acetate, 20 mM tris acetate pH 7.9, 1 mM cobalt chloride), and (3) 3'-O-azidomethyl TTP at 37 °C for 60 min. The 3'-blocked nucleotide triphosphate was at a concentration of 1 mM and the DNA initiator at 200 nM. The reaction was then stopped with EDTA and exposed to 30 µM DBCO-PEG4-TAMRA for 30 min. The reaction was then analysed in the FAM (solid line) and TAMRA (dotted line) channels by capillary electrophoresis, as shown in Figure 3. The successful addition of DBCO-PEG4-TAMRA (illustrated by a reaction schematic presented in Figure 3) is evident by the appearance of a DNA species with (1) higher retention compared to the N peak and (2) comparable signal in both the FAM and TAMRA emission channels, which indicates that the DNA species contains both FAM and TAMRA fluorophores (N+1 peak in Figure 3).

The cycloaddition of an azide to an alkyne results in a 1,2,3-triazole that is stable to reducing agents, such as β-mercaptoethanol (BME), DTT, and TCEP. Thus, treatment of a nucleic acid strand containing a 3'-O-azidomethyl protecting group with an alkyne species, such as a DBCO analogue, will render the nucleic acid strand irreversibly blocked in a DNA synthesis method and incapable of further extension.

## Claims

1. A method of nucleic acid synthesis, which comprises the steps of:
(a) providing an initiator sequence;
(b) adding a 3'-O-azidomethyl blocked nucleotide triphosphate to said initiator sequence in the presence of terminal deoxynucleotidyl transferase (TdT) or a functional equivalent or fragment thereof which retains the ability to catalyse the addition of a nucleotide onto the 3'-end of a nucleic acid strand,
(c) removal of TdT;
(d) cleaving the 3'-O-azidomethyl group from the 3'-O-azidomethyl blocked nucleotide triphosphate in the presence of a cleaving agent;
(e) removal of the cleaving agent; and
(f) adding a capping group to any uncleaved 3'-O-azidomethyl groups via a 1,3-dipolar cycloaddition reaction.

2. The method as defined in claim 1 wherein the capping group is a dipolarophile consisting in an alkyne, such as a strained alkyne.

3. The method as defined in claim 2, wherein the dipolarophile is dibenzocyclooctyne-amine.

4. The method as defined in any one of claims 1 to 3 wherein the 1,3-dipolar cycloaddition reaction of step (f) comprises an uncatalysed cycloaddition reaction.

5. The method as defined in any one of claims 1 to 3, wherein the 1,3-dipolar cycloaddition reaction of step (f) comprises a cycloaddition reaction catalysed by a copper or ruthenium-based catalyst.

6. The method as defined in any one of claims 1 to 5, wherein the capping group comprises one half of a binding pair, such as biotin.

7. The method as defined in any one of claims 1 to 6, wherein the capping group comprises a fluorine containing moiety.

8. The method as defined in any one of claims 1 to 7, wherein the capping group comprises a fluorescent moiety.

9. The method as defined in any one of claims 1 to 8, wherein the 3'-O-azidomethyl blocked nucleotide triphosphate is selected from a compound of formula (I), (II), (III) or (IV): 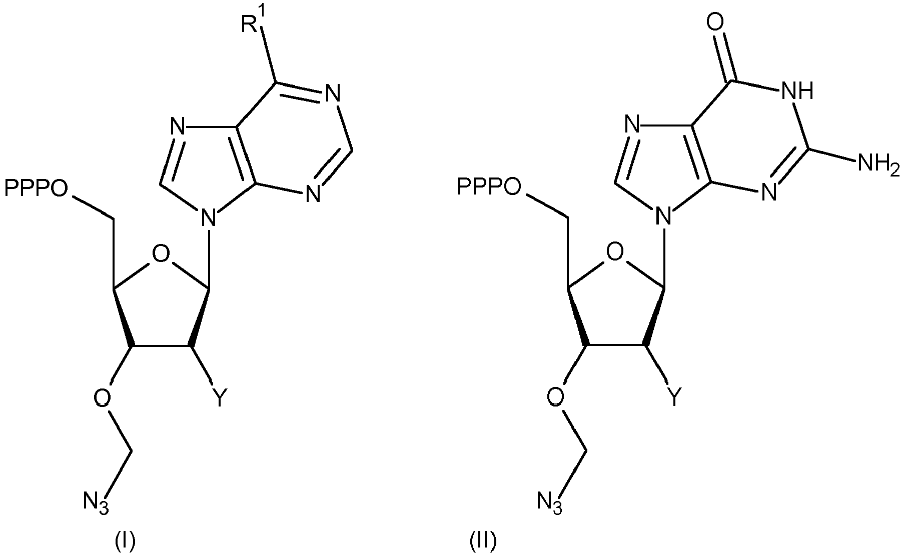 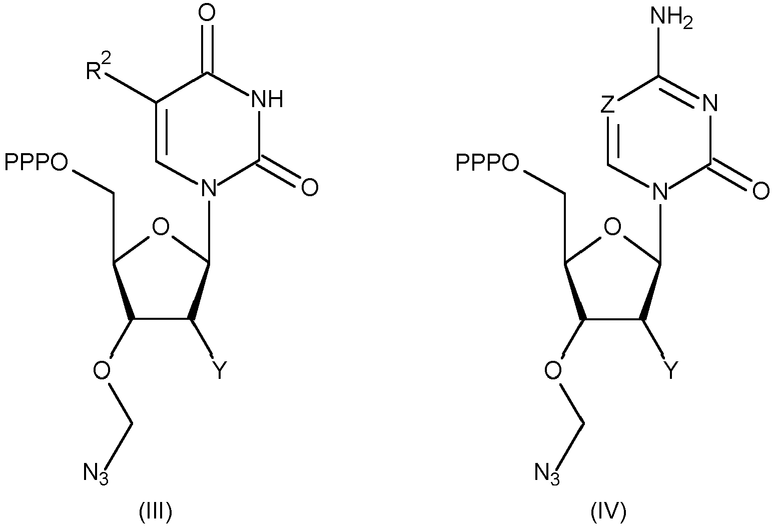 wherein
R¹ represents NR^{a}R^{b}, wherein R^{a} and R^{b} independently represent hydrogen or C₁₋₆ alkyl;
R² represents hydrogen, C₁₋₆ alkoxy, COH, COOH or C₁₋₆ alkyl optionally substituted by one or more OH or COOH groups;
Y represents hydrogen, hydroxyl or halogen; and
Z represents CR⁴ or N, wherein R⁴ represents hydrogen, C₁₋₆alkoxy, COH, COOH or C₁₋₆ alkyl optionally substituted by one or more OH or COOH groups.

10. The method as defined in any one of claims 1 to 9, wherein the terminal deoxynucleotidyl transferase (TdT) enzyme comprises: an amino acid sequence selected from any one of SEQ ID NOS: 1 to 5 and 8 or a functional equivalent or fragment thereof which retains the ability to catalyse the addition of a nucleotide onto the 3'-end of a nucleic acid strand having at least 20% sequence homology to said amino acid sequence; or a non-natural, mutated derivative of SEQ ID NO: 6.

11. The method as defined in any one of claims 1 to 10, wherein greater than 1 nucleotide is added by repeating steps (b) to (f).

12. Use of a kit to reduce mutations resulting from 3'-O-azidomethyl groups that are not deprotected during the deprotection step in a method of nucleic acid synthesis, wherein said kit comprises a 3'-O-azidomethyl capping group as defined in any one of claims 1 to 8, optionally in combination with one or more components selected from: terminal deoxynucleotidyl transferase (TdT) or a functional equivalent or fragment thereof which retains the ability to catalyse the addition of a nucleotide onto the 3'-end of a nucleic acid strand, an initiator sequence, one or more 3'-blocked nucleotide triphosphates, inorganic pyrophosphatase, such as purified, recombinant inorganic pyrophosphatase from *Saccharomyces cerevisiae,* a cleaving agent, an extension solution, a wash solution and/or a cleaving solution; further optionally together with instructions for use of the kit in accordance with the method as defined in any one of claims 1 to 11.

## Patentansprüche

1. Verfahren zur Nukleinsäuresynthese, das die folgenden Schritte umfasst:
(a) Bereitstellen einer Initiatorsequenz;
(b) Hinzufügen eines 3'-O-Azidomethyl-blockierten Nukleotidtriphosphats zu der Initiatorsequenz in Gegenwart von terminaler Desoxynukleotidyltransferase (TdT) oder eines funktionellen Äquivalents oder Fragments davon, welches weiterhin die Fähigkeit aufweist, das Hinzufügen eines Nukleotids am 3'-Ende eines Nukleinsäurestrangs zu katalysieren;
(c) Entfernung der TdT;
(d) Abspalten der 3'-O-Azidomethyl-Gruppe von dem 3'-O-Azidomethyl-blockierten Nukleotidtriphosphat in Gegenwart eines Spaltungsmittels;
(e) Entfernung des Spaltungsmittel; und
(f) Hinzufügen einer Verkappungsgruppe zu allen nicht abgespalteten 3'-O-Azidomethyl-Gruppen über eine 1,3-dipolare Cycloadditionsreaktion.

2. Verfahren nach Anspruch 1, wobei es sich bei der Verkappungsgruppe um ein Dipolarophil handelt, das aus einem Alkin, wie etwa einem gespannten Alkin besteht.

3. Verfahren nach Anspruch 2, wobei es sich bei dem Dipolarophil um Dibenzocyclooctyn-amin handelt.

4. Verfahren nach einem der Ansprüche 1 bis 3, wobei die 1,3-dipolare Cycloadditionsreaktion aus Schritt (f) eine unkatalysierte Cycloadditionsreaktion umfasst.

5. Verfahren nach einem der Ansprüche 1 bis 3, wobei die 1,3-dipolare Cycloadditionsreaktion aus Schritt (f) eine durch einen Katalysator auf Kupfer- oder Rutheniumbasis katalysierte Cycloadditionsreaktion umfasst.

6. Verfahren nach einem der Ansprüche 1 bis 5, wobei die Verkappungsgruppe eine Hälfte eines Bindungspaares, wie etwa Biotin, umfasst.

7. Verfahren nach einem der Ansprüche 1 bis 6, wobei die Verkappungsgruppe einen fluorhaltigen Rest umfasst.

8. Verfahren nach einem der Ansprüche 1 bis 7, wobei die Verkappungsgruppe einen fluoreszierenden Rest umfasst.

9. Verfahren nach einem der Ansprüche 1 bis 8, wobei das 3'-O-Azidomethyl-blockierte Nukleotidtriphosphat aus einer Verbindung der Formel (I), (II), (III) oder (IV) ausgewählt ist: 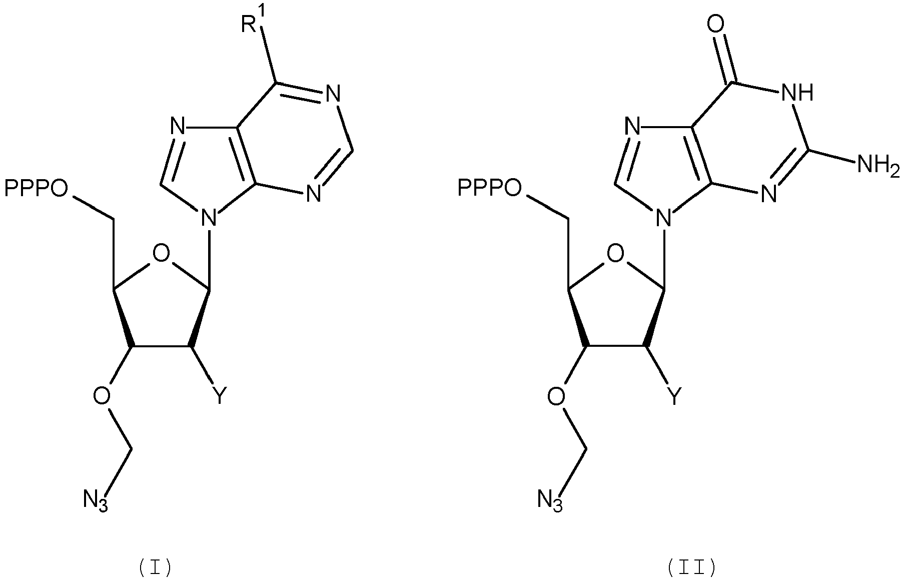 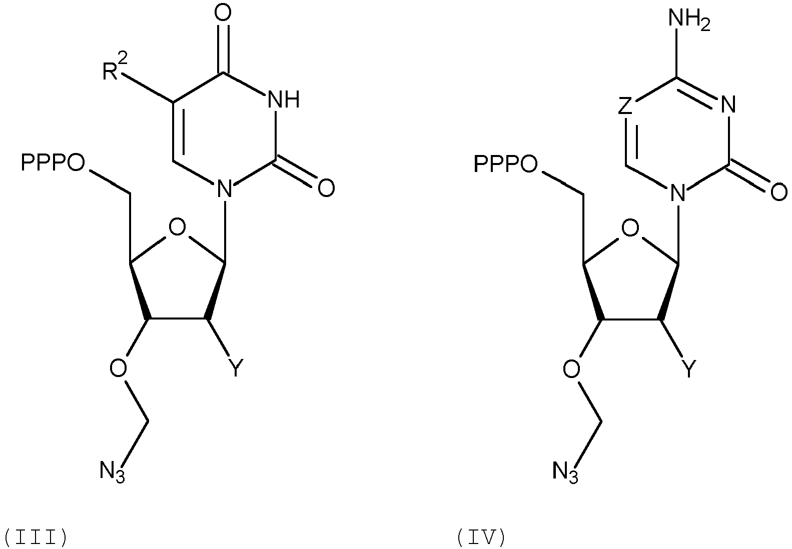 wobei
R¹ für NR^{a}R^{b} steht, wobei R^{a} und R^{b} unabhängig voneinander für Wasserstoff oder C₁₋₆-Alkyl stehen;
R² für Wasserstoff, C₁₋₆-Alkoxy, COH, COOH oder C₁₋₆ Alkyl steht, optional substituiert durch eine oder mehrere OH- oder COOH-Gruppen;
Y für Wasserstoff, Hydroxyl oder Halogen steht; und
Z für CR⁴ oder N steht, wobei R⁴ für Wasserstoff, C₁₋₆-Alkoxy, COH, COOH oder C-₁₋₆-Alkyl steht, optional substituiert durch eine oder mehrere OH- oder COOH-Gruppen.

10. Verfahren nach einem der Ansprüche 1 bis 9, wobei das Enzym der terminalen Desoxynucleotidyltransferase (TdT) Folgendes umfasst: eine Aminosäuresequenz, die aus einer der folgenden SEQ ID NOS ausgewählt ist: 1 bis 5 und 8 oder einem funktionellen Äquivalent oder Fragment davon, das weiterhin die Fähigkeit aufweist, das Hinzufügen eines Nukleotids am 3'-Ende eines Nukleinsäurestrangs zu katalysieren, der eine Sequenzhomologie mit der Aminosäuresequenz von mindestens 20 % aufweist; oder ein nicht natürliches, mutiertes Derivat der SEQ ID NO: 6.

11. Verfahren nach einem der Ansprüche 1 bis 10, wobei durch Wiederholen der Schritte (b) bis (f) mehr als 1 Nukleotid hinzugefügt wird.

12. Verwendung eines Kits zum Reduzieren von Mutationen, die durch 3'-O-Azidomethyl-Gruppen entstehen, die während des Entschützungsschritts in einem Verfahren zur Nukleinsäuresynthese nicht entschützt werden, wobei das Kit eine 3'-O-Azidomethyl-Verkappungsgruppe nach einem der Ansprüche 1 bis 8 umfasst, optional in Kombination mit einer oder mehreren aus den folgenden ausgewählten Komponenten: terminaler Desoxynukleotidyltransferase (TdT) oder einem funktionellen Äquivalent oder Fragment davon, welches weiterhin die Fähigkeit aufweist, das Hinzufügen eines Nukleotids am 3'-Ende eines Nukleinsäurestrangs zu katalysieren, einer Initiatorsequenz, einem oder mehreren 3'-blockierten Nukleotidtriphosphaten, anorganischer Pyrophosphatase, wie etwa gereinigter, rekombinanter anorganischer Pyrophosphatase von *Saccharomyces cerevisiae,* einem Spaltungsmittel, einer Verlängerungslösung, einer Waschlösung und/oder einer Spaltungslösung; ferner optional zusammen mit Anweisungen zur Verwendung des Kits gemäß dem Verfahren nach einem der Ansprüche 1 bis 11.

## Revendications

1. Procédé de synthèse d'acide nucléique, qui comprend les étapes :
(a) de fourniture d'une séquence d'initiateurs ;
(b) d'addition d'un 3'-O-azidométhylnucléotide triphosphate bloqué à ladite séquence d'initiateurs en présence de la désoxynucléotidyl-transférase terminale (TdT) ou d'un équivalent fonctionnel ou d'un fragment de celle-ci qui conserve la capacité à catalyser l'addition d'un nucléotide sur l'extrémité 3' d'un brin d'acide nucléique,
(c) d'élimination de TdT ;
(d) de clivage du groupe 3'-O-azidométhyle du 3'-O-azidométhylnucléotide triphosphate bloqué en présence d'un agent de clivage ;
(e) d'élimination de l'agent de clivage ; et
(f) d'addition d'un groupe de coiffage à tout groupe 3'-O-azidométhyle non clivé par l'intermédiaire d'une réaction de cycloaddition 1,3-dipolaire.

2. Procédé selon la revendication 1, dans lequel le groupe de coiffage est un dipolarophile constitué d'un alcyne, tel qu'un alcyne contraint.

3. Procédé selon la revendication 2, dans lequel le dipolarophile est la dibenzocyclooctyne-amine.

4. Procédé selon l'une quelconque des revendications 1 à 3, dans lequel la réaction de cycloaddition 1,3-dipolaire de l'étape (f) comprend une réaction de cycloaddition non catalysée.

5. Procédé selon l'une quelconque des revendications 1 à 3, dans lequel la réaction de cycloaddition 1,3-dipolaire de l'étape (f) comprend une réaction de cycloaddition non catalysée par un catalyseur à base de cuivre ou de ruthénium.

6. Procédé selon l'une quelconque des revendications 1 à 5, dans lequel le groupe de coiffage comprend la moitié d'une paire de liaison, telle que la biotine.

7. Procédé selon l'une quelconque des revendications 1 à 6, dans lequel le groupe de coiffage comprend un fragment contenant du fluor.

8. Procédé selon l'une quelconque des revendications 1 à 7, dans lequel le groupe de coiffage comprend un fragment fluorescent.

9. Procédé selon l'une quelconque des revendications 1 à 8, dans lequel le 3'-O-azidométhylnucléotide triphosphate bloqué est choisi parmi un composé de formule (I), (II), (III) ou (IV) : 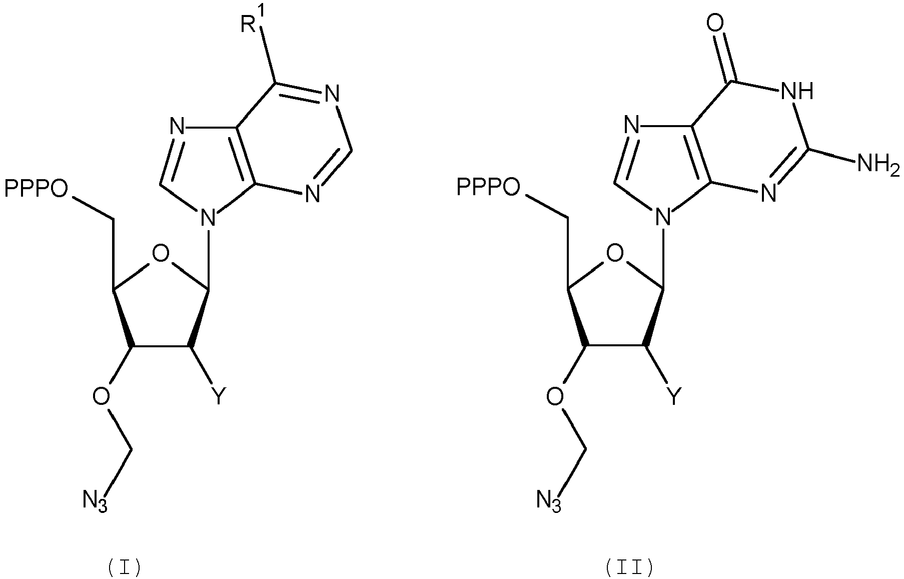 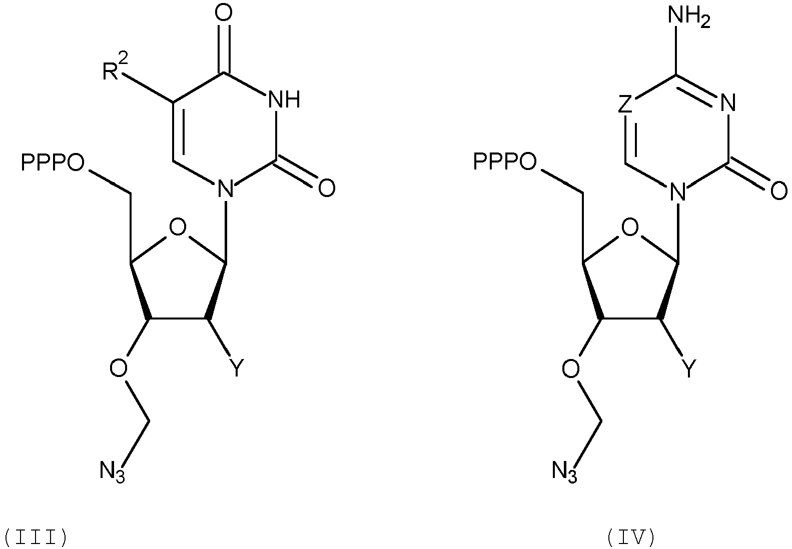 dans lequel
R¹ représente NR^{a}R^{b}, dans lequel R^{a} et R^{b} représentent indépendamment l'hydrogène ou un groupe alkyle en C₁₋₆ ;
R² représente l'hydrogène, un groupe alcoxy en C₁₋₆, COH, COOH ou alkyle en C₁₋₆ éventuellement substitué par un ou plusieurs groupes OH ou COOH ;
Y représente l'hydrogène, un groupe hydroxyle ou halogène ; et
Z représente CR⁴ ou N, dans lequel R⁴ représente l'hydrogène, un groupe alcoxy en C₁₋₆, COH, COOH ou alkyle en C₁₋₆ éventuellement substitué par un ou plusieurs groupes OH ou COOH.

10. Procédé selon l'une quelconque des revendications 1 à 9, dans lequel l'enzyme désoxynucléotidyl-transférase terminale (TdT) comprend : une séquence d'acides aminés choisie parmi l'un quelconque des SEQ ID N° : 1 à 5 et 8 ou un équivalent fonctionnel ou un fragment de celle-ci qui conserve la capacité à catalyser l'addition d'un nucléotide sur l'extrémité 3' d'un brin d'acide nucléique ayant au moins 20 % d'homologie de séquence avec ladite séquence d'acides aminés ; ou un dérivé non naturel et muté de SEQ ID N° : 6.

11. Procédé selon l'une quelconque des revendications 1 à 10, dans lequel plus de 1 nucléotide est ajouté en répétant les étapes (b) à (f).

12. Utilisation d'un kit pour réduire les mutations résultant de groupes 3'-O-azidométhyle qui ne sont pas déprotégés pendant l'étape de déprotection dans un procédé de synthèse d'acide nucléique, dans laquelle ledit kit comprend un groupe de coiffage 3'-O-azidométhyl tel que défini dans l'une quelconque des revendications 1 à 8, éventuellement en combinaison avec un ou plusieurs composants choisis parmi :
la désoxynucléotidyl-transférase terminale (TdT) ou un équivalent fonctionnel ou un fragment de celle-ci qui conserve la capacité à catalyser l'addition d'un nucléotide sur l'extrémité 3' d'un brin d'acide nucléique, une séquence d'initiateurs, un ou plusieurs triphosphates de nucléotides bloqués en 3', la pyrophosphatase inorganique, telle que la pyrophosphatase inorganique recombinante purifiée à partir de *Saccharomyces cerevisiae,* un agent de clivage, une solution d'extension, une solution de lavage et/ou une solution de clivage ; en outre éventuellement avec des instructions pour l'utilisation du kit selon le procédé tel que défini dans l'une quelconque des revendications 1 à 11.
